# EUROPEAN PATENT APPLICATION

(11) **EP 4 559 483 A1**
(43) Date of publication of application: **28.05.2025**
(21) Application number: 23843041.7
(22) Date of filing: 20.07.2023
(51) Int. Cl.: A61K 47/36, A61K 9/10, A61K 38/12, A61K 38/13, C08B 37/08

(54) **HYALURONIC ACID DERIVATIVE DRUG COMPOSITION AND DRUG COMPOSITION**

(30) Priority: 20.07.2022 JP 2022115840; 09.09.2022 JP 2022143474
(71) Applicant: Asahi Kasei Kabushiki Kaisha, Tokyo 1000006 (JP)
(72) Inventor: NAKAI Takashi, Tokyo 100-0006 (JP); YABUUCHI Kohei, Tokyo 100-0006 (JP)
(74) Representative: Hoffmann Eitle
(86) International application number: PCT/JP2023/026609
(87) International publication number: WO 2024/019118

(57) **Abstract**

A hyaluronic acid derivative pharmaceutical composition includes an active ingredient suspended in an aqueous carrier containing a hyaluronic acid derivative, in which the active ingredient is a fine particle having a molecular weight of 100 g/mol or greater and 8000 g/mol or less. It is preferable that the fine particle is a microparticle or a nanoparticle.

## Description

### TECHNICAL FIELD

The present invention relates to a hyaluronic acid derivative pharmaceutical composition and a pharmaceutical composition.

Priorities are claimed on Japanese Patent Application No. 2022-115840 filed on July 20, 2022 and Japanese Patent Application No. 2022-143474 filed on September 9, 2022, the contents of which are incorporated herein.

### BACKGROUND ART

In recent years, biopharmaceuticals, which are pharmaceutical products containing proteins, peptides, or nucleic acids as active ingredients, have been put into practical use, and the number of biopharmaceuticals has been increasing year by year. Biopharmaceuticals can satisfy unmet medical needs that have not been satisfied by low-molecular-weight drugs.

However, biopharmaceuticals have problems in that the biopharmaceuticals are difficult to be absorbed from the digestive tract, the mucous membrane, and the like, and are unstable in the body and have a short half-life in blood. Therefore, the biopharmaceuticals require frequent administration by injection, which is a heavy burden on both patients and medical personnel. Therefore, there is a demand for a drug base material (a sustained-release drug delivery system base material) that can encapsulate a biopharmaceutical and gradually release an active ingredient in vivo without impairing the pharmacological activity.

From such a background, Patent Document 1 suggests a sustained-release drug delivery system base material formed of a hyaluronic acid derivative having excellent safety.

The hyaluronic acid derivative disclosed in Patent Document 1 can spontaneously associate in an aqueous solution, and can efficiently enclose a drug, particularly a biopharmaceutical, while maintaining the biological activity thereof. In this manner, the derivative aggregates at a physiological saline concentration (or is dispersed even at a physiological saline concentration) and has satisfactory blood retention properties. This hyaluronic acid derivative can be used as a carrier which can efficiently enclose a large amount of a drug while maintaining pharmacological activity, a blood sustained-release carrier having excellent blood retention properties, and a targeting carrier, particularly in a case where a biopharmaceutical is used as an active ingredient, and the hyaluronic acid derivative is also considered to be used as a local (for example, subcutaneous) sustained-release carrier which can continuously allow sustained-release of a drug.

### Citation List

### Patent Document

Patent Document 1: PCT International Publication No. WO2010/053140

### SUMMARY OF INVENTION

### Technical Problem

A sustained-release drug base material is required to maintain the concentration of the active ingredient in vivo for a longer period of time and to gradually release the active ingredient. In addition, it is required to suppress an initial release amount abnormality (initial burst).

The present invention has been made in consideration of the above-described circumstances, and an object thereof is to provide a hyaluronic acid derivative pharmaceutical composition and a pharmaceutical composition which maintain the concentration of an active ingredient in vivo for a long period of time and reduce then initial release amount abnormality (initial burst).

### Solution to Problem

That is, the present invention includes the following aspects.
[1] A hyaluronic acid derivative pharmaceutical composition including: an active ingredient suspended in an aqueous carrier containing a hyaluronic acid derivative, in which the active ingredient is a fine particle having a molecular weight of 100 g/mol or greater and 8000 g/mol or less.
[2] The hyaluronic acid derivative pharmaceutical composition according to [1], in which the fine particle is a microparticle or a nanoparticle.
[3] The hyaluronic acid derivative pharmaceutical composition according to [1] or [2], in which the hyaluronic acid derivative has one or more repeating units represented by General Formula (I). 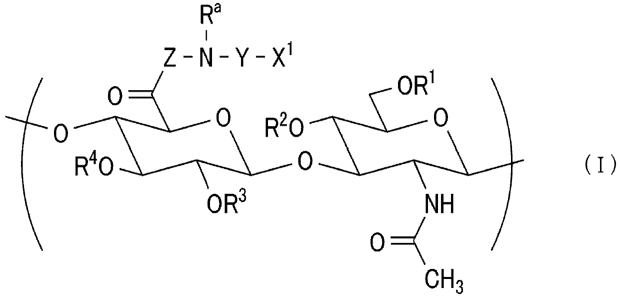
   (In the formula, R¹, R², R³, and R⁴ each independently represent a group selected from the group consisting of a hydrogen atom, C₁₋₆ alkyl, formyl, and C₁₋₆ alkylcarbonyl. Z represents a direct bond or a peptide linker formed of 2 or more and 30 or less of any amino acid residues.
   X¹ represents a group selected from the group consisting of a group represented by -NR^{b}-R, -NR^{b}-COO-R, -NR^{b}-CO-R, -NR^{b}-CO-NR^{c}-R, -COO-R, -O-COO-R, -S-R, - CO-Y^{a}-S-R, -O-CO-Y^{b}-S-R, -NR^{b}-CO-Y^{b}-S-R, and -S-S-R.
   R^{a}, R^{b}, and R^{c} each independently represent a group selected from the group consisting of a hydrogen atom, C₁₋₂₀ alkyl, amino C₂₋₂₀ alkyl, and hydroxy C₂₋₂₀ alkyl. A group selected from the group consisting of -O- and -NR^{f}- may be inserted into an alkyl moiety of each of R^{a}, R^{b}, and R^{c}.
   R^{f} represents a group selected from the group consisting of a hydrogen atom, C₁₋₁₂ alkyl, amino C₂₋₁₂ alkyl, and hydroxy C₂₋₁₂ alkyl. A group selected from the group consisting of -O- and -NH- may be inserted into an alkyl moiety of R^{f}.
   R represents a hydrophobic group or a hydrophilic group.
   Y represents C₂₋₃₀ alkylene or -(CH₂CH₂O)ₘ-CH₂CH₂-. Here, a group selected from the group consisting of -O-, -NR^{g}-, and -S-S- may be inserted into alkylene of Y.
   R^{g} represents a group selected from the group consisting of a hydrogen atom, C₁₋₂₀ alkyl, amino C₂₋₂₀ alkyl, and hydroxy C₂₋₂₀ alkyl. A group selected from the group consisting of -O- and -NH- may be inserted into an alkyl moiety of R^{g}.
   Y^{a} represents C₁₋₅ alkylene.
   Y^{b} represents C₂₋₈ alkylene or C₂₋₈ alkenylene.
   m represents an integer of 1 or greater and 100 or less.)
[4] The hyaluronic acid derivative pharmaceutical composition according to [3], in which R represents a hydrophobic group.
[5] The hyaluronic acid derivative pharmaceutical composition according to [4], in which the hydrophobic group is a cholesteryl group.
[6] The hyaluronic acid derivative pharmaceutical composition according to [1] or [2], in which the hyaluronic acid derivative is precipitated or salted out in vivo or under a physiological condition.
[7] The hyaluronic acid derivative pharmaceutical composition according to any one of [1] to [6], in which the active ingredient is a poorly water-soluble drug.
[8] The hyaluronic acid derivative pharmaceutical composition according to any one of [1] to [7], in which the active ingredient is a cyclic peptide.
[9] The hyaluronic acid derivative pharmaceutical composition according to [8], in which the cyclic peptide contains 4 to 49 amino acids in terms of a cyclic size.
[10] The hyaluronic acid derivative pharmaceutical composition according to [8] or [9], in which the cyclic peptide contains an unnatural amino acid.
[11] The hyaluronic acid derivative pharmaceutical composition according to any one of [8] to [10], in which the number of kinds of amino acids constituting the cyclic peptide is 8 or more and 20 or less.
[12] The hyaluronic acid derivative pharmaceutical composition according to any one of [1] to [11], in which the hyaluronic acid derivative pharmaceutical composition is administered subcutaneously, intramuscularly, intraspinally, intraarticularly, intracerebrally, intravitreally, or to an ocular surface, a nose, a lung, or a mouth as an administration site.
[13] The hyaluronic acid derivative pharmaceutical composition according to any one of [1] to [12], in which an administration method is injection, eye drop, or application.

### Advantageous Effects of Invention

According to the present invention, it is possible to provide a hyaluronic acid derivative pharmaceutical composition and a pharmaceutical composition which maintain the concentration of an active ingredient in vivo for a long period of time and reduce the initial release amount abnormality (initial burst).

### BRIEF DESCRIPTION OF DRAWINGS

[FIG. 1] FIG. 1 shows an X-ray powder diffraction peak of cyclosporin.
[FIG. 2] FIG. 2 is a graph showing a result of a change in blood plasma concentration of cyclosporin during administration of a cyclosporin suspension preparation.
[FIG. 3] FIG. 3 is a graph showing a result of a change in blood plasma concentration of cyclosporin during administration of a cyclosporin suspension preparation.
[FIG. 4] FIG. 4 is a photographic view showing a result of precipitation evaluation in a vitreous body.
[FIG. 5] FIG. 5 is a graph showing a result of a change in blood plasma concentration of cyclosporin during administration of a cyclosporin suspension preparation.
[FIG. 6] FIG. 6 is a graph showing a result of a change in blood plasma concentration of cyclosporin during administration of a cyclosporin suspension preparation.
[FIG. 7] FIG. 7 is a graph showing the results of the change in the concentration of cyclosporin in blood plasma at the time of the administration of the cyclosporin suspension preparation.
[FIG. 8] FIG. 8 is a graph showing a result of a change in blood plasma concentration of cyclosporin during administration of a cyclosporin suspension preparation.
[FIG. 9] FIG. 9 is a graph showing a result of a change in blood plasma concentration of cyclosporin during administration of a cyclosporin suspension preparation.
[FIG. 10] FIG. 10 is a graph showing a result of a change in blood plasma concentration of cyclosporin during administration of a cyclosporin suspension preparation.

### DESCRIPTION OF EMBODIMENTS

Hereinafter, embodiments of the present invention (hereinafter, referred to as "present embodiment") will be described in detail, but the present invention is not limited thereto, and various modifications can be made without departing from the scope of the present invention.

Hereinafter, the terms used in the present specification will be described.

The term "C₁₋₂₀ alkyl" used in the present specification means a linear or branched alkyl group having 1 or more and 20 or less carbon atoms, and includes, for example, "C₁₋₄ alkyl" such as methyl, ethyl, n-propyl, iso-propyl, n-butyl, sec-butyl, isobutyl, or tert-butyl, and further includes n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, or 2-ethylbutyl. The C₁₋₂₀ alkyl also includes a C₁₋₁₂ alkyl having 1 or more and 12 or less carbon atoms and a C₁₋₆ alkyl group having 1 or more and 6 or less carbon atoms.

The term "C₁₋₆ alkylcarbonyl" used in the present specification means an alkylcarbonyl group in which the alkyl moiety is the above-described C₁₋₆ alkyl, and includes, for example, "C₁₋₄ alkylcarbonyl" such as acetyl, propionyl, n-propylcarbonyl, iso-propylcarbonyl, n-butylcarbonyl, sec-butylcarbonyl, iso-butylcarbonyl, or tert-butylcarbonyl.

The term "amino C₂₋₂₀ alkyl" used in the present specification means a linear or branched alkyl having 2 or more and 20 or less carbon atoms, which has an amino group as a substituent, and for example, the amino group may be positioned on a carbon atom at a terminal of the alkyl group. The amino C₂₋₂₀ alkyl also includes an amino C₂₋₁₂ alkyl having 2 or more and 12 or less carbon atoms.

The term "hydroxy C₂₋₂₀ alkyl" used in the present specification means a linear or branched alkyl group having 2 or more and 20 or less carbon atoms, which has a hydroxy group as a substituent, and for example, the hydroxy group may be positioned on a carbon atom at a terminal of the alkyl group. The hydroxy C₂₋₂₀ alkyl also includes a hydroxy C₂₋₁₂ alkyl having 2 or more and 12 or less carbon atoms.

The term "C₂₋₃₀ alkylene" used in the present specification means a linear or branched divalent saturated hydrocarbon group having 2 or more and 30 or less carbon atoms, and includes, for example, ethylene and propylene, and C₂₋₂₀ alkylene having 2 or more and 20 or less carbon atoms, C₂₋₈ alkylene having 2 or more and 8 or less carbon atoms, and a group "-(CH₂)ₙ-" (here, n represents 2 or greater and 30 or less, preferably represents 2 or greater and 20 or less, and more preferably represents 2 or greater and 15 or less).

The term "C₁₋₅ alkylene" used in the present specification means a linear or branched divalent saturated hydrocarbon group having 1 or more and 5 or less carbon atoms, and includes, for example, methylene, ethylene, and propylene.

The term "C₂₋₈ alkenylene" described in the present specification means a divalent saturated hydrocarbon group which is linear or branched and has 2 or more and 8 or less carbon atoms and includes one or more double bonds, and includes, for example, -CH=CH-, -C(CH₃)=CH-, 2-butene-1,4-diyl, hepta-2,4-dien-1,6-diyl, and octa-2,4,6-triene-1,8-diyl. In a case where geometric isomers are present, each of the isomers and a mixture thereof are also included.

### <Hyaluronic acid derivative pharmaceutical composition>

The present embodiment relates to a hyaluronic acid derivative pharmaceutical composition containing an active ingredient suspended in an aqueous carrier containing a hyaluronic acid derivative.

### <<Aqueous carrier containing hyaluronic acid derivative>>

The aqueous carrier containing a hyaluronic acid derivative in the present specification is an aqueous carrier containing a hyaluronic acid derivative described below.

In the present specification, "aqueous carrier" is a pharmaceutically acceptable carrier, and examples thereof include a substance approved as a pharmaceutical additive for an injection preparation by the Pharmaceuticals and Medical Devices Agency (PMDA) or the Food and Drug Administration (FDA), or a substance listed as a pharmaceutical additive for an injection preparation in the Japanese Pharmacopoeia, the United States Pharmacopeia, or other generally recognized pharmacopoeias for use in animals, more specifically, humans.

Specifically, the aqueous carrier is preferably a sterile liquid, and may be, for example, water, oil, or an emulsion (for example, oil/water or a water/oil emulsion).

The water includes water, water for injection, a physiological saline solution, a phosphate buffer solution, a sucrose-containing phosphate buffer solution, a sucrose aqueous solution, a glucose aqueous solution, a maltose aqueous solution, a trehalose aqueous solution, a glycerol aqueous solution, a polyethylene glycol-containing aqueous solution, a polysorbate-containing aqueous solution, a poloxamer-containing aqueous solution, a cyclodextrin-containing aqueous solution, a polyvinylpyrrolidone-containing aqueous solution, and a carboxymethyl cellulose-containing aqueous solution.

The oil includes petroleum, animal oils, plant oils, and synthetic oils, for example, peanut oil, soybean oil, mineral oil, and sesame oil.

In the present embodiment, the aqueous carrier is preferably one or two or more selected from the group consisting of water, water for injection, a physiological saline solution, an aqueous buffer solution, a phosphate buffer solution, a sucrose-containing phosphate buffer solution, a sucrose aqueous solution, a glucose aqueous solution, and a glycerol aqueous solution. Among these, the aqueous carrier is more preferably water, a phosphate buffer solution, a sucrose-containing phosphate buffer solution, or a sucrose aqueous solution.

The aqueous carrier may contain a solvent, such as a diluent, an adjuvant, an excipient, a stabilizer, an isotonic agent, a buffer, a pH adjuster, a preservative, a fungicide or an antibacterial agent, or a thickener, which are administered together with a compound.

In the present specification, the expression "contains an active ingredient suspended in an aqueous carrier containing a hyaluronic acid derivative" denotes a state in which fine particles of the active ingredient are not dissolved in the aqueous carrier containing a hyaluronic acid derivative, and the particles are dispersed and floated in the aqueous carrier containing a hyaluronic acid derivative.

In the present specification, "suspension" means suspension in a broad sense, and indicates that a dispersoid is dispersed in a dispersion medium.

One aspect of the suspension indicates a dispersion system in which solid particles are dispersed in a liquid.

In the present specification, the dispersoid may be a solid, a liquid, or a gas, and the dispersion medium may be a liquid. In addition, liquid-liquid phase separation in which the dispersion medium and the dispersoid which is a liquid are dispersed without being mixed with each other is also regarded as one aspect of suspension.

The fine particles of the active ingredient may be uniformly or non-uniformly suspended in the aqueous carrier, but are preferably uniformly suspended.

Whether or not the fine particles of the active ingredient are suspended in the aqueous carrier containing a hyaluronic acid derivative does not depend only on the upper limit of the solubility of the aqueous carrier, and the particle size of the fine particles also affects the suspension. In a case where all the particles are instantaneously (for example, within 1 second) sedimented due to a large size of the particles and are not floated in the aqueous carrier, it does not correspond to "suspended in the aqueous carrier". From the viewpoint of more stably being "suspended in the aqueous carrier", the particle size of the fine particles is preferably in a range of 1 nm to 1000 µm, more preferably in a range of 5 nm to 100 µm, still more preferably in a range of 10 nm to 10 µm, even still more preferably in a range of 50 nm to 10 µm, even still more preferably in a range of 50 nm to 5 µm, even still more preferably in a range of 50 nm to 1 µm, and most preferably in a range of 50 nm to 500 nm.

In addition, the period during which the active ingredient is "suspended in the aqueous carrier" is preferably, for example, 1 year or longer as a pharmaceutical product. However, even in a case where the active ingredient is sedimented once, the active ingredient is uniformly dispersed again by stirring the aqueous carrier before administration, and in a case where the active ingredient is in a state of "being suspended in the aqueous carrier", it is determined that "the active ingredient is contained in the suspended aqueous carrier containing the hyaluronic acid derivative".

The active ingredient being in the form of a solid can be determined by, for example, visual observation or microscopic observation. The solid can maintain a certain shape different from a liquid or a gas. In addition, the solid can also be determined from a property of being difficult to deform.

In the present embodiment, whether or not the aqueous carrier containing a hyaluronic acid derivative contains a suspended active ingredient can be specifically determined by a turbidity test method, which is a general test method described in the Japanese Pharmacopoeia. The turbidity test method can be determined by a visual observation method, a transmitted light measurement method, a scattered light measurement method, or a photoelectric photometry method such as a transmission scattering method. In a case of a pharmaceutical in a suspended state, for example, the pharmaceutical is described in the attached document. The description is not particularly limited, but the pharmaceutical is described as a suspension, a suspension injection, an aqueous suspension injection, or a suspension agent eye drop.

It is more preferable that the fine particles of the active ingredient are not sedimented in the aqueous carrier.

In a case where the fine particles can be floated in the aqueous carrier while maintaining the state in which the fine particles are dispersed, the hyaluronic acid derivative is gelated or precipitated at a physiological saline concentration in a case of being administered in vivo, and the fine particles are retained and carried in the gel of the hyaluronic acid derivative or the precipitate, whereby the fine particles are stably retained without being diffused from the administration site. As a result, the amount of the active ingredient and the surface area are locally maintained, stable long-term sustained release is exhibited, and the initial release amount abnormality can be reduced.

It is preferable that the fine particles of the active ingredient are uniformly maintained in the aqueous carrier without sedimentation, and in a case where the hyaluronic acid derivative is gelated and precipitated at a physiological saline concentration, the fine particles are precipitated together with the hyaluronic acid derivative.

For example, in a case where only the fine particles of the active ingredient are administered in vivo without using the hyaluronic acid derivative, the initial release amount abnormality is likely to occur because the fine particles are diffused in vivo after administration, and the long-term sustained release is not exhibited. In addition, in a case where the dispersion state of the fine particles is not maintained and the fine particles are aggregated, the surface area is reduced. As a result, the dissolution rate of the active ingredient is decreased, and the expected drug efficacy cannot be obtained because the blood concentration does not reach a sufficient level. **In** addition, in a case where the fine particles are administered without using the hyaluronic acid derivative, the fine particles do not remain at the administration site, and thus the presence state of the fine particles varies for each administration, which causes variation.

As a technology of the related art, a method of forming a complex of a hyaluronic acid derivative and an active ingredient is known. "Forming a complex of a hyaluronic acid derivative and an active ingredient" means that the active ingredient is retained in a physical crosslinking domain of the hyaluronic acid derivative due to a hydrophobic interaction.

In a case where a complex of the hyaluronic acid derivative and the active ingredient are formed, the amount of the active ingredient to be carried is limited to 1 part by mass to 100 parts by mass at most with respect to 100 parts by mass of the hyaluronic acid derivative. Meanwhile, the hyaluronic acid derivative pharmaceutical composition of the present invention is not limited as long as the active ingredient is in a state of being suspended in the aqueous carrier.

In a case where a complex of the hyaluronic acid derivative and the active ingredient are formed, the active ingredient is solubilized and is in a liquid state. Therefore, even in a case where the hyaluronic acid derivative is gelated or precipitated after administration in vivo, the active ingredient is relatively quickly released and migrates into the blood. In addition, depending on the interaction of the active ingredients, the precipitation property of the hyaluronic acid derivative is decreased in a case of administration in vivo, and for example, in a case where the hyaluronic acid derivative in the pharmaceutical composition is not completely precipitated at 5%, the active ingredient forming a complex with the hyaluronic acid derivative rapidly migrates into the blood, that is, the rapid migration is a major cause of the initial burst. Meanwhile, in the hyaluronic acid derivative pharmaceutical composition of the present invention, since the active ingredient is in a solid state and the precipitation performance in a case where the hyaluronic acid derivative is administered in vivo is not impaired, even in a case where the hyaluronic acid derivative in the pharmaceutical composition is not completely precipitated at 5%, the active ingredient is slowly dissolved and absorbed due to the solubility limit, and thus the initial burst can be maximally suppressed.

### <<Hyaluronic acid derivative>>

The hyaluronic acid derivative used in the present embodiment is preferably a hyaluronic acid derivative having one or more repeating units represented by General Formula (I). 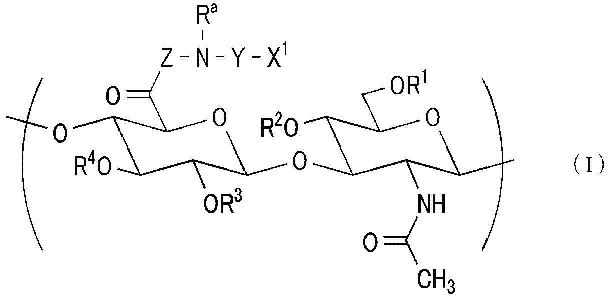 (In the formula, R¹, R², R³, and R⁴ each independently represent a group selected from the group consisting of a hydrogen atom, C₁₋₆ alkyl, formyl, and C₁₋₆ alkylcarbonyl.

Z represents a direct bond or a peptide linker formed of 2 or more and 30 or less of any amino acid residues.

X¹ represents a group selected from the group consisting of a group represented by -NR^{b}-R, -NR^{b}-COO-R, -NR^{b}-CO-R, -NR^{b}-CO-NR^{c}-R, -COO-R, -O-COO-R, -S-R, - CO-Y^{a}-S-R, -O-CO-Y^{b}-S-R, -NR^{b}-CO-Y^{b}-S-R, and -S-S-R.

R^{a}, R^{b}, and R^{c} each independently represent a group selected from the group consisting of a hydrogen atom, C₁₋₂₀ alkyl, amino C₂₋₂₀ alkyl, and hydroxy C₂₋₂₀ alkyl. Here, a group selected from the group consisting of -O- and -NR^{f}- may be inserted into the alkyl moiety of each of R^{a}, R^{b}, and R^{c}.

R^{f} represents a group selected from the group consisting of a hydrogen atom, C₁₋₁₂ alkyl, amino C₂₋₁₂ alkyl, and hydroxy C₂₋₁₂ alkyl. A group selected from the group consisting of -O- and -NH- may be inserted into an alkyl moiety of R^{f}.

R represents a hydrophobic group or a hydrophilic group.

Y represents C₂₋₃₀ alkylene or -(CH₂CH₂O)ₘ-CH₂CH₂-. Here, a group selected from the group consisting of -O-, -NR^{g}-, and -S-S- may be inserted into alkylene of Y.

R^{g} represents a group selected from the group consisting of a hydrogen atom, C₁₋₂₀ alkyl, amino C₂₋₂₀ alkyl, and hydroxy C₂₋₂₀ alkyl. A group selected from the group consisting of -O- and -NH- may be inserted into an alkyl moiety of R^{g}.

Y^{a} represents C₁₋₅ alkylene.

Y^{b} represents C₂₋₈ alkylene or C₂₋₈ alkenylene.

m represents an integer of 1 or greater and 100 or less.)

The hyaluronic acid derivative preferably includes a hyaluronic acid derivative having one or more repeating units represented by General Formula (Ia) (hereinafter, also referred to as "repeating unit (Ia)"). 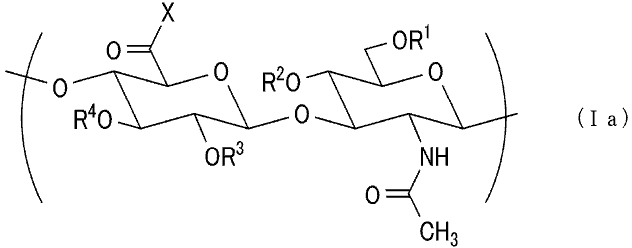 **(In** the formula, R¹, R², R³, and R⁴ each independently represent a group selected from the group consisting of a hydrogen atom, C₁₋₆ alkyl, formyl, and C₁₋₆ alkylcarbonyl. X represents a hydrophobic group represented by -NR^{a}-Y-NR^{b}-COO-R. R^{a} and R^{b} each independently represent a group selected from the group consisting of a hydrogen atom and C₁₋₆ alkyl. R represents a hydrophobic group or a hydrophilic group. Y represents C₂₋₃₀ alkylene or -(CH₂CH₂O)ₘ-CH₂CH₂-, where m represents an integer of 1 or greater and 100 or less)

Here, in a case where the hyaluronic acid derivative has two or more of the repeating unit (I) or two or more of the repeating units (Ia), the repeating units may be the same as or different from each other.

The hyaluronic acid derivative may be modified at a position other than the repeating unit (I) or the repeating unit (Ia), for example, the hydroxy group may be converted into -O(C₁₋₆ alkyl), -O(formyl), -O(C₁₋₆ alkylcarbonyl), or the like, and the carboxy group may be converted into an amide or an ester or form a salt.

### [Repeating unit (I)]

A group "-Z-N(R^{a})Y-X¹" in General Formula (I) includes a group selected from the group consisting of groups represented by the following formula: -NH-(CH₂)_{mz}-NH-R; -NH-(CH₂)_{mz}-NH-COO-R; -NH-(CH₂CH₂O)ₘ-CH₂CH₂-NH-COO-R; -NH-(CH₂)_{mz}-COO-R; -NH-(CH₂CH₂O)ₘ-CH₂CH₂-COO-R, -NH-(CH₂)_{mz}-O-COO-R; -NH-(CH₂CH₂O)ₘ-CH₂CH₂-O-COO-R, -NH-(CH₂)_{mz}-S-R; -NH-(CH₂CH₂O)ₘ-CH₂CH₂-S-R; - NH-(CH₂)_{mz}-O-CO-CH(R⁸)-CH₂-S-R; -NH-(CH₂)_{mz}-NHCO-CH(R⁸)-CH₂-S-R; -NH-(CH₂CH₂O)ₘ-CH₂CH₂-NHCO-CH(R⁸)-CH₂-S-R; -NH-(CH₂CH₂O)ₘ-CH₂CH₂-O-CO-CH(R⁸)-CH₂-S-R; -NH-(CH₂)_{mz}-S-S-R; and -Z-NR^{a}-Y-NR^{b}-COO-R (here, mz represents an integer of 2 or greater and 30 or less, R⁸ represents a hydrogen atom or a methyl group, and R and m are as defined in the present specification).

As the group, a group selected from the group consisting of -NH-(CH₂)_{mz}-NH-COO-R; -NH-(CH₂CH₂O)ₘ-CH₂CH₂-NH-COO-R; and -NH-(CH₂)_{mz}-S-S-R (here, mz, R, and m are as defined in the present specification) is preferable.

### (Z)

In General Formula (I), it is preferable that Z represents a direct bond. In addition, in another aspect, in a case where Z represents a peptide linker, it is preferable that X¹ represents -NR^{b}-COO-R. Further, in another aspect, Z may represent a peptide linker represented by -NH-[CH(-Z^{a})-CONH]ₙ₋₁-CH(-Z^{a})-CO-, where n represents an integer of 2 or greater and 30 or less, and Z^{a}'s each independently represent a substituent in an α-amino acid represented by H₂N-CH(-Z^{a})-COOH. The peptide linker is bonded to a carboxy group of a glucuronic acid moiety at the N-terminal and is bonded to a group-N(-R^{a})-Y-X¹ at the C-terminal. Examples of the amino acid that can be used as the amino acid residue of the peptide linker include natural type (L-type) amino acids such as α-amino acids such as alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine, and D-forms thereof, and all α-amino acids including the synthesized amino acids can be used. That is, examples of Z^{a} include -CH₃, H₂NC(NH)NH(CH₂)₃-, and H₂NCOCH₂-. In addition, n pieces of Z's may be the same as or different from each other. n represents an integer of 2 or greater and 30 or less, preferably 2 or greater and 10 or less, and more preferably 2 or greater and 4 or less. Preferred examples of the peptide linker include -Gly-Phe-Leu-Gly-, -Asn-Phe-Phe-, -Phe-Phe-, and Phe-Gly-.

As the term "linker" herein, any peptide linker or synthetic compound linker that can be introduced by genetic engineering can be used, but in the hyaluronic acid derivative (A) used in the present embodiment, a peptide linker is preferable. The length of the peptide linker is not particularly limited, and can be appropriately selected by those skilled in the art according to the purpose, but the length is preferably 2 amino acids or more (the upper limit is not particularly limited, but is usually 30 amino acids or less and preferably 20 amino acids or less) and particularly preferably 15 amino acids. In the peptide linker contained in the hyaluronic acid derivative (A), peptide linkers having the same length or peptide linkers having different lengths may be used.

### (Y)

In General Formula (I), it is preferable that Y represents a group selected from the group consisting -(CH₂)ₙ₁- and -(CH₂CH₂O)ₘ₁-CH₂CH₂- (here, n1 represents an integer of 2 or greater and 20 or less, preferably an integer of 2 or greater and 15 or less, more preferably an integer of 2 or greater and 12 or less, and still more preferably an integer of 2 or greater and 6 or less. m1 represents an integer of 1 or greater and 4 or less). Specifically, -(CH₂)₂-, -(CH₂)₆-, -(CH₂)₈-, -(CH₂)₁₂-, or -(CH₂CH₂O)₂-CH₂CH₂- is preferable. In addition, from the viewpoint of realizing high solubility in pure water or a low salt concentration and exhibiting high precipitate forming ability at a physiological saline concentration, Y represents preferably a group selected from the group consisting of -(CH₂)₂-, -(CH₂)₆-, -(CH₂)₈-, and -(CH₂)₁₂- and more preferably -(CH₂)₆-.

Y may represent, for example, -CH₂CH₂O-CH₂CH₂-S-S-CH₂CH₂O-CH₂CH₂-, - (CH₂CH₂O)₂-CH₂CH₂-S-S-CH₂CH₂O-CH₂CH₂-, -CH₂CH₂O-CH₂CH₂-S-S-(CH₂CH₂O)₂-CH₂CH₂-, or -(CH₂CH₂O)₂-CH₂CH₂-S-S-(CH₂CH₂O)₂-CH₂CH₂-.

### (Y^{a})

It is preferable that Y^{a} represents -CH₂- or -CH₂-CH₂-.

### (Y^{b})

Y^{b} represents preferably -CH₂-CH₂-, -CH(CH₃)CH₂-, 2-butene-1,4-diyl, hepta-2,4-diene-1,6-diyl, or octa-2,4,6-triene-1,8-diyl and more preferably -CH₂-CH₂- or - CH(CH₃)CH₂-.

Specific examples of the group "-Z-N(R^{a})Y-X¹" include -NH-(CH₂)₂-NH-CO-cholesteryl, -NH-(CH₂)₄-NH-(CH₂)₃-NH-(CH₂)₃-NH-COO-cholesteryl, -NH-(CH₂)₃-NH-(CH₂)₄-NH-(CH₂)₃-NH-COO-cholesteryl, -NH-(CH₂)₄-NH-(CH₂)₃-NH-COO-cholesteryl, -NH-(CH₂)₄-N(-(CH₂)₃-NH₂)-COO-cholesteryl, -NH-(CH₂)₃-NH-(CH₂)₄-N(-(CH₂)₃-NH₂)-COO-cholesteryl, -NH-(CH₂)₃-NH-(CH₂)₄-N(-(CH₂)₃-NH-(CH₂)₃-NH₂)-COO-cholesteryl, -NH-(CH₂)₃-NH-(CH₂)₄-N(-(CH₂)₃-NH₂)-CO-NH-cholesteryl, -NH-(CH₂)₃-NH-(CH₂)₄-N(-(CH₂)₃-NH₂)-CO-cholesteryl, and -NH-(CH₂)₃-NH-(CH₂)₄-N(-(CH₂)₃-NH₂)-cholesteryl. As the preferable group "-Z-N(R^{a})Y-X¹", R^{a}, R^{b}, and R^{c} each represent a hydrogen atom, Y represents linear C₂₋₃₀ alkylene or -(CH₂CH₂O)ₘ-CH₂CH₂-, Y^{a} represents linear C₁₋₅ alkylene, or Y^{b} represents linear C₂₋₈ alkylene or linear C₂₋₈ alkenylene.

### (R)

R represents a hydrophilic group or a hydrophobic group and preferably a hydrophobic group.

Examples of the hydrophilic group include a derivative having a polyethylene glycol skeleton, a derivative having a polyethyleneimine skeleton, a derivative having a polylisine skeleton, a derivative having a polyallylamine skeleton, a derivative having a natural amino acid skeleton, a derivative having an unnatural amino acid skeleton, and a derivative having a polydimethylsiloxane skeleton.

As the hydrophobic group, a steryl group is preferable.

The term "steryl group" used in the present specification is not particularly limited as long as the group has a steroid skeleton. Here, specific examples of the steroid include cholesterol, cholestanol, campesterol, ergostanol, stigmasterol, coprostanol, stigmasterol, sitosterol, lanosterol, ergosterol, simianerol, bile acid, testosterone, estradiol, progesterone, cortisol, cortisone, aldosterone, corticosterone, and deoxycorticosterone. Examples of the steryl group include a cholesteryl group, a stigmasteryl group, a lanosterol group, and an ergosterol group. Among these, a cholesteryl group (particularly, a cholesta-5-en-3β-yl group) is preferable.

### [Repeating unit (Ia)]

In General Formula (Ia), X represents preferably -NH-(CH₂)₂-NH-COO-cholesteryl, -NH-(CH₂)₆-NH-COO-cholesteryl, -NH-(CH₂)₁₂-NH-COO-cholesteryl, or - NH-(CH₂CH₂O)₂-CH₂CH₂-NH-COO-cholesteryl and more preferably -NH-(CH₂)₂-NH-COO-cholesteryl, -NH-(CH₂)₆-NH-COO-cholesteryl, or -NH-(CH₂CH₂O)₂-CH₂CH₂-NH-COO-cholesteryl.

The hyaluronic acid derivative can further have a repeating unit represented by General Formula (II) (hereinafter, also referred to as "repeating unit (II)") in addition to the repeating unit (I). 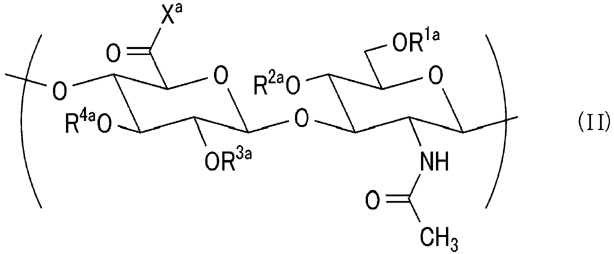 (In the formula, R^{1a}, R^{2a}, R^{3a}, and R^{4a} each independently represent a group selected from the group consisting of a hydrogen atom, C₁₋₆ alkyl, formyl, and C₁₋₆ alkylcarbonyl. X^{a} represents a group selected from the group consisting of hydroxy and -O-Q⁺. Q⁺ represents a counter cation.)

Here, in a case where the hyaluronic acid derivative has two or more repeating units (II), the repeating units may be the same as or different from each other.

In another aspect, the hyaluronic acid derivative may be a hyaluronic acid derivative substantially having the repeating unit (I), the repeating unit (Ia), and the repeating unit (II).

### [Repeating unit (II)]

In General Formula (II), Q⁺ is not particularly limited as long as Q⁺ represents a counter cation that forms a salt with a carboxy group in water, and in a case of being divalent or higher, the counter cation forms a salt with a plurality of carboxy groups according to the valence. Examples of the counter cation include metal ions such as a lithium ion, a sodium ion, a rubidium ion, a cesium ion, a magnesium ion, and a calcium ion; and an ammonium ion represented by Formula: N⁺R^{j}R^{k}R^{l}R^{m} (in the formula, R^{j}, R^{k}, R^{l}, and R^{m} are each independently selected from the group consisting of a hydrogen atom and C₁₋₆ alkyl). Among these, the Q⁺ represents preferably a sodium ion, a potassium ion, or a tetraalkylammonium ion (for example, a tetra-n-butylammonium ion). R^{j}, R^{k}, R^{l}, and R^{m} represent preferably the same group selected from the group consisting of C₁₋₆ alkyls and preferably an n-butyl group.

It is preferable that all of R¹, R², R³, R⁴, R^{1a}, R^{2a}, R^{3a}, and R^{4a} represent a hydrogen atom. In addition, it is preferable that both R^{a} and R^{b} represent a hydrogen atom.

Among these, the hyaluronic acid derivative is preferably a hyaluronic acid derivative substantially formed of the repeating unit (I) and the repeating unit (II). In the hyaluronic acid derivative, for example, 80% or greater, preferably 90% or greater, and more preferably 95% or greater of the repeating units of the disaccharide consisting of D-glucuronic acid and N-acetyl-D-glucosamine contained in the derivative are the repeating unit (I) and the repeating unit (II). The hyaluronic acid derivative may be formed of only the repeating unit (I) and the repeating unit (II).

In the hyaluronic acid derivative of the present embodiment, the steryl group introduction rate (hereinafter, also simply referred to as "steryl group introduction rate") with respect to the repeating unit of the disaccharide derived from the hyaluronic acid derivative is preferably 0.1% or greater and less than 50%, more preferably 5% or greater and less than 45%, still more preferably 10% or greater and 40% or less, and particularly preferably 15% or greater and 35% or less. In a case where the steryl group introduction rate is in the above-described ranges, the stability of the preparation is improved, and the hyaluronic acid derivative pharmaceutical composition containing fine particles of the active ingredient in the pharmaceutical composition is aggregated and precipitated at a physiological saline concentration, thereby enabling sustained release of the drug.

The introduction rate of the steryl group can be measured by ¹H-NMR measurement. That is, the steryl group introduction rate can be calculated based on the following equation using the integral value of the peak derived from the steryl group of the hyaluronic acid derivative in the ¹H-NMR spectrum of the hyaluronic acid derivative component and the integral value of the peak (COCH₃, 1.6 ppm or greater and 2.0 ppm or less, 3H) derived from the acetyl group of N-acetyl-D-glucosamine contained in the hyaluronic acid derivative. In addition, in the equation, n_{H} represents the number of hydrogen atoms corresponding to the peak. Specifically, the measurement can be carried out by, for example, a method described in examples described below. [Introduction rate of steryl group] (%) = [(peak integral value derived from steryl group × 3/nH)/(peak integral value derived from acetyl group of N-acetyl-D-glucosamine)] × 100

The weight-average molecular weight of the hyaluronic acid derivative is not particularly limited, but from the viewpoint of increasing the number of introduced steryl groups per molecule of the hyaluronic acid derivative to form a complex with the active ingredient, and from the viewpoint of increasing the entanglement of molecules to increase the retention properties in blood, a hyaluronic acid derivative having a relatively large molecular weight is preferable.

The weight-average molecular weight of such a hyaluronic acid derivative is preferably 4,000 (4 k) or greater and 1,000,000 (1,000k) or less, more preferably 5k or greater and 500k or less, still more preferably 7k or greater and 300k or less, and particularly preferably 7k or greater and 100k or less.

**In** a case where the weight-average molecular weight of the hyaluronic acid derivative is greater than or equal to the above-described lower limits, the entanglement of molecules can be further enhanced, and the retention properties in blood can be further enhanced. Meanwhile, in a case where the weight-average molecular weight of the hyaluronic acid derivative is less than or equal to the above-described upper limits, an increase in viscosity can be suppressed, and the hyaluronic acid derivative at a higher concentration can be dissolved in the pharmaceutical composition. The weight-average molecular weight of the hyaluronic acid derivative can usually be adjusted by using a raw material having a corresponding molecular weight.

More specifically, from the viewpoint of viscosity and dispersibility, the weight-average molecular weight of the hyaluronic acid derivative is preferably 100 k or less, more preferably 50 k or less, particularly preferably 20 k or less, and most preferably 15 k or less. The molecular weight thereof is in units of Da.

From the viewpoint of producing a hyaluronic acid derivative, the weight-average molecular weight thereof is preferably 4 k to 20 k, particularly preferably 6 k to 16 k, and most preferably 8 k to 12 k.

From the viewpoint that the hyaluronic acid derivative strongly exhibits the properties of hyaluronic acid, for example, the molecular weight of the hyaluronic acid derivative is preferably 100 k or greater, particularly preferably 200 k or more, and most preferably 300 k or greater in order for the hyaluronic acid derivative to be strongly recognized by the CD44 receptor.

From the viewpoint of controlling the viscosity and the precipitation rate of the hyaluronic acid derivative, the molecular weight of the hyaluronic acid derivative is preferably 8 k or greater, particularly preferably 20 k or greater, and most preferably 30 k or greater.

The term "molecular weight of the hyaluronic acid derivative" described herein is a weight-average molecular weight determined by a size exclusion chromatography multi-angle light scattering detector (SEC-MALS).

### [Method of producing hyaluronic acid derivative]

The hyaluronic acid derivative can be produced, for example, by the method described in PCT International Publication No. WO2010/053140 in a case where R represents a hyaluronic acid derivative into which a hydrophobic group has been introduced. In a case where R represents a hydrophilic group, the hyaluronic acid derivative may be produced by introducing an amino group into a terminal of the derivative having a hydrophilic group by any method, and then performing a condensation reaction with a carboxyl (-COOH or -COONa) group of hyaluronic acid using a condensing agent such as DMT-MM (4-(4,6-dimethoxy-1,3,5-triazin-2-yl)-4-methylmorpholinium chloride).

In addition, in a case where the compound intended to be introduced has a hydroxy (-OH) group at the terminal, the compound may be produced by dewater condensation using any condensing agent. In addition, a chemical bond may be formed by reacting a hydroxy group of hyaluronic acid with a functional group of an introduction group. Examples of the introduction method other than the condensing agent include an enzymatic reaction, dewater condensation by heat, and a method using a metal catalyst.

In the present specification, it is preferable that the hyaluronic acid derivative is precipitated or salted out in vivo or under physiological conditions.

Whether or not the hyaluronic acid derivative is precipitated or is salted out in vivo or under physiological conditions can be confirmed by the following method.

First, the hyaluronic acid derivative dissolved at a desired concentration is added to a phosphate buffer solution or BSA-containing PBS. Thereafter, the mixture is incubated at 37°C and centrifuged, and in a case where the solid-liquid separation can be carried out, it is determined that the hyaluronic acid derivative is precipitated or salted out with physiological saline. The incubation time may be optionally set.

The concentration of the phosphate buffer solution to be used is, for example, a final concentration of 1 × PBS or PBS containing 0.8% BSA.

Centrifugal conditions in the case of centrifugation can be appropriately changed, and an example thereof is 9800 g.

In one aspect of the present invention, the proportion of the precipitated and salted-out hyaluronic acid derivative under physiological conditions can be quantified by adding 150 µL of 4 × PBS to 450 µL of the hyaluronic acid derivative dissolved at a desired concentration, incubating the mixture under the above-described conditions, and quantifying the supernatant of the hyaluronic acid derivative sedimented by centrifugation by size exclusion chromatography (SEC).

In one aspect of the present invention, the precipitated and salt-out hyaluronic acid derivative under the physiological salt conditions can be confirmed by actually administering the hyaluronic acid derivative in vivo to confirm the precipitation or by administering the hyaluronic acid derivative to a collected tissue to confirm the precipitation.

The proportion of the precipitate is preferably 10% to 100%, preferably 40% or greater, 50% or greater, and 60% or greater, more preferably 70% or greater, still more preferably 80% or greater, and most preferably 90% or greater with respect to the total amount of the dissolved hyaluronic acid derivative.

In a case of using the hyaluronic acid derivative that is precipitated or salted out in vivo or under physiological conditions, the hyaluronic acid derivative is gelated after administration in vivo, and thus the aggregation or diffusion of fine particles of the active ingredient contained in the pharmaceutical composition is suppressed. Therefore, it is easy to provide a hyaluronic acid derivative pharmaceutical composition in which the concentration of the active ingredient is maintained in vivo for a long period of time without variation and the initial release amount abnormality (initial burst) is reduced.

### <<Active ingredient>>

The active ingredient is not particularly limited as long as the active ingredient is fine particles having a molecular weight of 100 g/mol or greater and 8000 g/mol or less.

The molecular weight of the fine particles is preferably 200 g/mol or greater and 8000 g/mol or less, more preferably 200 g/mol or greater and 5000 g/mol or less, still more preferably 300 g/mol or greater and 4000 g/mol or less, even still more preferably 500 g/mol or greater and 4000 g/mol or less, particularly preferably 700 g/mol or greater and 3000 g/mol or less, even particularly preferably 900 g/mol or greater and 3000 g/mol or less, still more preferably 1000 g/mol or greater and 3000 g/mol or less, and most preferably 1000 g/mol or greater and 2000 g/mol or less.

In a case where the molecular weight of the fine particles is in the above-described ranges, the fine particles can form nanoparticles or microparticles and can freely float in the aqueous carrier without sedimentation.

In the present specification, the molecular weight of the fine particles of the active ingredient is a value calculated from the molecular formula of the compound.

In the present embodiment, the fine particles are preferably microparticles or nanoparticles.

The microparticles denote particles having a particle diameter of 1 µm or greater and less than 1000 µm, which are measured by a laser diffraction method.

The nanoparticles denote particles having a particle diameter of 1 nm or greater and less than 1000 nm, which are measured by a dynamic light scattering method (abbreviated as DLS).

The active ingredient that can be used in the present embodiment is not particularly limited as long as the active ingredient is the above-described fine particles, and examples thereof include a low-molecular-weight compound, a medium-molecular-weight compound, a pharmaceutically active peptide or protein, and a nucleic acid. Among these, a low-molecular-weight compound, a medium-molecular-weight compound, a pharmaceutically active peptide, or a protein is more preferable. A low-molecular-weight compound, a medium-molecular-weight compound, or a pharmaceutically active peptide is more preferable. The medium-molecular-weight compound may be a cyclic peptide.

The fine particles may be crystalline or amorphous.

The diseases to which the pharmaceutical composition of the present embodiment is applied are not particularly limited, and the pharmaceutical composition can be widely used for the prevention or treatment of diseases known at present or diseases that will be found in the future. The disease may be either a chronic disease or an acute disease. Examples of diseases known at present include cancer, infectious disease, immune disease, inflammatory laxity, allergic disease, skin disease, hypertension, diabetes, nervous disease, hereditary disease, cardiovascular disease, cerebrovascular disease, respiratory disease, eye disease, ear disease, bone and joint disease, and pain.

**In** a case where the active ingredient in the present invention is a poorly water-soluble drug, the present invention can be more effectively used.

The poorly water-soluble drug means a drug classified as slightly easily soluble, slightly poorly soluble, poorly soluble, extremely poorly soluble, or hardly soluble among drugs which are described as extremely easily soluble, easily soluble, slightly easily soluble, slightly poorly soluble, poorly soluble, extremely poorly soluble, or hardly soluble in the terms indicating solubility in the 17th revised Japanese Pharmacopoeia.

Specific examples of the active ingredient include poorly water-soluble drugs having a solubility of 1 mg/mL or less in water.

Even in the case of such a poorly water-soluble drug, the pharmaceutical composition of the present invention can be administered at a high dose without using an organic solvent and without reducing the amount of a highly toxic surfactant used or without using the highly toxic surfactant at all.

In the present embodiment, examples of the poorly water-soluble drug include poorly water-soluble peptides. The poorly water-soluble peptide may have an acidic amino acid, a basic amino acid, or a neutral amino acid. The pH of the aqueous carrier may be appropriately selected in consideration of the isoelectric point.

In the present embodiment, the active ingredient is preferably a cyclic peptide. As the cyclic size, a cyclic peptide formed of 4 to 49 amino acids is preferable, a cyclic peptide formed of 6 to 30 amino acids is more preferable, and a cyclic peptide formed of 8 to 25 amino acids is most preferable.

The cyclic peptide may contain a natural amino acid or an unnatural amino acid. Among these, it is preferable that the cyclic peptide contains an unnatural amino acid.

The number of kinds of amino acids constituting the cyclic peptide is preferably 8 or more and 20 or less.

### [Low-molecular-weight compound]

The low-molecular-weight compound is a compound having a molecular weight of less than about 500, and examples thereof include an anticancer agent (for example, an alkylating agent, a metabolite antagonist, an alkaloid, and the like), an immunosuppressant, an anti-inflammatory agent (a steroid agent, a non-steroid anti-inflammatory agent, and the like), an anti-rheumatic agent, and an antibacterial agent (a β-lactam antibiotic, an aminoglycoside antibiotic, a macrolide antibiotic, a tetracycline antibiotic, a new quinolone antibiotic, a sulfa drug, and the like).

**In** addition, in a case where the above-described hyaluronic acid derivative has a steryl group as the active ingredient, a hydrophobic ingredient, that is, a poorly water-soluble ingredient can also be preferably used since the interaction with the steryl group can be sufficiently exhibited. The term "poorly water-soluble" means that the amount of water of 30 mL or greater is required to dissolve 1 g of a solute, according to the 17th revised Japanese Pharmacopoeia.

Examples of the poorly water-soluble and solid active ingredient include antipyretic and analgesic agents such as acetaminophen, ibuprofen, benzoic acid, ethenzamide, caffeine, camphor, quinine, calcium gluconate, dimethylcaprol, sulfamine, theophylline, theopromine, riboflavin, mephenesine, phenobarbital, aminophylline, thioacetazone, quercetin, rutin, salicylic acid, theophylline sodium salt, pyrapital, quinine hydrochloride, irgacure, dikitoxin, griseofulvin, and phenacetin; neurological medicines, sedative-hypnotic drugs, muscle relaxants, antihypertensive agents, antihistamines; antibiotics such as acetylspiramycin, ampicillin, erythromycin, xylatamycin, chloramphenicol, triazetylolerythromycin, nystatin, colistin sulfate; steroid hormones such as methyltestosterone, methylandrosteronedione, progesterone, estradiol benzoate, ethinylestradiol, deoxycorticosterone acetate, cortisone acetate, hydrocortisone, hydrocortisone acetate, and bredonizolone; non-steroidal yolk hormone agents such as dienestrol, hexestrol, diethylstilbestrol, diethylstilbestrol dibrohydionate, and chlorotrianisene; and other pharmaceutical active ingredients described in "Japanese Pharmacopoeia", "Japanese Pharmaceutical Codex ", "USP", "NF", and "EP". Such active ingredients may be used alone or a combination of two or more kinds selected from the above-described active ingredients may be used.

The active ingredient may be a poorly water-soluble oily or liquid ingredient. Examples of the poorly water-soluble oily or liquid active ingredients include, for example, vitamins such as teprenone, indomethacin pharnesil, menatetrenone, phytomenadione, vitamin A oil, phenipentol, vitamin D, and vitamin E, highly unsaturated fatty acids such as DHA (docosahexaenoic acid), EPA (eicosapentaenoic acid), and cod liver oil, coenzyme Q, and oil-soluble flavoring agents such as orange oil, lemon oil, and peppermint oil, which are described in the "Japanese Pharmacopoeia", " Japanese Pharmaceutical Codex", "USP", "NF", and "EP". Vitamin E has various homologues and derivatives, and the vitamin E is not particularly limited as long as it is in a liquid state at room temperature. Examples of the vitamin E include dl-α-tocopherol, dl-α-tocopherol acetate, d-α-tocopherol, and d-α-tocopherol acetate. Such active ingredients may be used alone or a combination of two or more kinds selected from the above-described active ingredients may be used.

The active ingredient may be a poorly water-soluble semi-solid active ingredient. Examples of the poorly water-soluble semi-solid active ingredient include a Chinese medicine or a crude drug extract such as earthwworm, kanzo, keihi, shakuyaku, buttonpi, kanokosou, sanshou, shoukyou, chinki, maou, nantengitsu, ouhi, onji, kikyou, shazenshi, shazenso, ishibaraso, seneka, baimo, uikyou, oubaku, uren, gajutsu, kamitsure, gentiana, goou, joudan, shazin, shoukyou, soujutsu, chouji, chinki, byakujutsu, chikusetsuninjin, ninjin, kakkontou, keishitou, kousosan, shikokeito, shoushikuto, shoukeiryoutou, bakumondoutou, hanboukutouboku, and maoutou; and oyster meat extract, propolis and propolis extract, and coenzyme Q. Such active ingredients may be used alone or a combination of two or more kinds selected from the above-described active ingredients may be used.

### [Medium-molecular-weight compound]

In the present specification, "medium-molecular-weight" refers to a peptide having a molecular weight of about 500 to 5,000, a macrolide compound, a natural product, or a derivative thereof, which is neither a low molecular weight (an organic compound having a molecular weight of about 500) nor a high molecular weight (a protein having a molecular weight of 10,000 or greater or the like). The peptide is preferably a peptide having a molecular weight of about 500 to 2000, that is, a linear or cyclic peptide having about 5 to 20 amino acid residues. The peptide is preferably a cyclic peptide, and the details thereof will be described below. The macrolide compound is a macrocyclic lactone, and is a general term for a compound having 12 or more ring members.

Examples thereof include FK506 and rapamycin.

### [Nucleic acid]

The nucleic acid includes DNA and RNA, and includes, for example, short interfering RNA (siRNA), double-stranded RNA (dsRNA), microRNA (miRNA), short hairpin RNA (shRNA), and a nucleic acid aptamer.

The active ingredient may be a Rho kinase inhibitor, an endothelin A receptor inhibitor, a transmembrane conductance regulator (CFTR) modulator, a TRPV1 inhibitor, an NK1 receptor inhibitor, a purine receptor inhibitor, an angiotensin receptor inhibitor, a peroxisome proliferator-activated receptor, a P2Y receptor inhibitor, a VEGF inhibitor, or the like, or may be an active ingredient having two inhibitory actions at the same time.

The content of the active ingredient in the pharmaceutical composition of the present embodiment depends on the structure of the active ingredient, but can be set to 0.001 parts by mass or greater and 10,000 parts by mass or less, preferably 0.1 parts by mass or greater and 8,000 parts by mass or less, more preferably 1.0 parts by mass or greater and 6,000.0 parts by mass or less, still more preferably 15 parts by mass or greater and 4,000.0 parts by mass or less, particularly preferably 30 parts by mass or greater and 2,000 parts by mass or less, and most preferably 100 parts by mass or greater and 1,000 parts by mass or less with respect to 100 parts by mass of the hyaluronic acid derivative.

Alternatively, in the pharmaceutical composition of the present embodiment, the content of the active ingredient is not particularly limited, but the content thereof is, for example, preferably 0.0001 parts by mass or greater and 30.0 parts by mass or less, more preferably 0.001 parts by mass or greater and 10 parts by mass or less, and still more preferably 0.01 parts by mass or greater and 3 parts by mass or less with respect to 100 parts by mass of the pharmaceutical composition.

### <<Other additives>>

The pharmaceutical composition according to the present embodiment can be administered alone or can be administered together with a pharmaceutically acceptable carrier according to means of the related art. In a case of being used in combination with a pharmaceutically acceptable carrier, for example, the above-described hyaluronic acid derivative and the above-described active ingredient, and as necessary, water or other physiologically acceptable liquids (for example, physiological saline, phosphate buffered physiological saline (PBS)), and the like may be mixed, and a physiologically acceptable buffer solution, an excipient, a vehicle, a preservative, a stabilizer, a binder, a lyophilization adjuvant, and the like may be included.

The pharmaceutical composition according to the present embodiment may contain one or a plurality of surfactants in the aqueous carrier. Among these, a nonionic surfactant is preferable, and the nonionic surfactant suitable for use in the preparation described in the present specification may be selected from, for example, polysorbate, poloxamer, a polyoxyethylene castor oil derivative (for example, Cremophor EL or Cremophor RH60), bile salt, lecithin, 12-hydroxystearic acid-polyethylene glycol copolymer (for example, Solutol HS 15), and chloroxylenol.

The pharmaceutical composition according to the present embodiment can contain one or a plurality of water-soluble polymers. The water-soluble polymer is a pharmaceutically acceptable polymer that can be dissolved or dispersed in water. The water-soluble polymer may have a function of, for example, reinforcing the viscosity of the suspension or stabilizing the active ingredient of the microparticles or the nanoparticles against particle aggregation or a potential harmful effect from other preparation ingredients. In the present embodiment, the water-soluble polymer suitable for use can be selected from, for example, plant gum such as an alginate, pectin, guar gum, or xanthan gum, chemical starch, polyvinylpyrrolidone (PVP), hypromellose (HPMC), methyl cellulose, and other cellulose derivatives such as sodium carboxymethyl cellulose, and hydroxypropyl cellulose. The pharmaceutical composition according to the present embodiment can contain a poloxamer such as poloxamer 188 as the water-soluble polymer. The poloxamer 188 is both a polymer and a surfactant. In another embodiment, the pharmaceutical composition of the present embodiment can contain povidone K17 as the water-soluble polymer.

The pharmaceutical composition of the present embodiment can contain one or more kinds of cellulose-based polymers, and according to WO2022/210784A1, it has been reported that an aqueous suspension agent having improved redispersibility can be provided. In addition, it is possible to provide an aqueous suspension agent in which the redispersibility is further improved by blending a zinc salt or a silver salt, and the zinc salt or the silver salt may be appropriately selected from a combination in consideration of compatibility with a drug or other additives. The term "zinc salt" refers to a salt (a compound of zinc and an organic acid or an inorganic acid) containing a zinc ion (Zn2+) as a constituent ion, or an oxide of zinc. Examples thereof include zinc halides such as zinc chloride, zinc bromide, and zinc fluoride, zinc sulfate, zinc acetate, zinc phosphate, zinc carbonate, zinc hydroxide, zinc citrate, zinc lactate, zinc gluconate, zinc oxide, and hydrates thereof. The term "silver salt" refers to a salt (a compound of silver and an organic acid or an inorganic acid) containing a silver ion (Ag+) as a constituent ion, or an oxide of silver. Examples thereof include silver nitrate, silver bromide, silver oxide, silver acetate, silver carbonate, silver citrate, silver lactate, silver phosphate, silver oxalate, silver thiosulfate, protein silver, and hydrates thereof.

Examples of the buffer solution include Tris, sodium phosphate, potassium phosphate, histidine, a borate buffer material, and citric acid.

The boric acid buffer is not particularly limited as long as it is pharmaceutically acceptable, and examples thereof include any one or both of boric acid and a salt of boric acid. The boric acid is not particularly limited as long as it is pharmaceutically acceptable, and examples thereof include orthoboric acid, metaboric acid, and tetraboric acid. The salt of boric acid is not particularly limited as long as it is pharmaceutically acceptable, and examples thereof include metal salts such as a sodium salt, a potassium salt, a calcium salt, a magnesium salt, and an aluminum salt; and organic amine salts such as triethylamine, triethanolamine, morpholine, piperazine, and pyrrolidine. The boric acid or the salt thereof may be used alone or a combination of two or more kinds thereof may be used. In addition, examples of a suitable aspect of the borate buffer agent include a combination of boric acid and borax.

The ratio of boric acid and borax in a case of being used in combination is not particularly limited. For example, the content of borax is 10 to 300 parts by mass, preferably 10 to 250 parts by mass, more preferably 30 to 100 parts by mass, and particularly preferably 40 to 60 parts by mass with respect to 100 parts by mass of boric acid.

Examples of the preservative include benzalkonium chloride, methyl parabenzoate, propyl parabenzoate, chlorobutanol, sorbic acid, and alkyl polyaminoethyl glycine.

Examples of the stabilizer include a sodium edetate hydrate and polyvinylpyrrolidone (povidone).

Examples of the isotonic agent include sodium chloride, glycerol, glucose, mannitol, sorbitol, and sucrose, which are commonly used. Among these, sucrose is preferable.

Examples of the antioxidant include ascorbic acid, an ascorbate such as sodium ascorbate, tocopherol, and a sulfite such as sodium sulfite, potassium sulfite, magnesium sulfite, calcium sulfite, sodium metabisulfite, potassium metabisulfite, magnesium metabisulfite, calcium metabisulfite, sodium pyrosulfite, potassium pyrosulfite, calcium pyrosulfite, sodium thiosulfate, or sodium hydrogen sulfite.

A cellulose derivative, polyethylene glycol (macrogol), or the like can also be contained as the viscosity adjuster.

The pharmaceutical composition of the present embodiment is not particularly limited as long as the composition is within the range accepted as a drug used as an artificial tear solution, but may contain aminoethylsulfonic acid, sodium chondroitin sulfate, potassium L-aspartate, magnesium L-aspartate, potassium **L-**aspartate/magnesium (a mixture of equivalent amounts), sodium hydrogen carbonate, sodium carbonate, potassium chloride, glucose, and propylene glycol.

A formulated product may be used as the pharmaceutical composition of the present embodiment. The preparation can be in the form of a solid, a semi-solid, or a liquid.

In a case of a solid, examples thereof include a powder, a granule, a tablet, a pellet, and a capsule. Among these, the solid is preferably a freeze-dried powder.

In a case of a semi-solid, examples thereof include a gel.

In a case of a liquid, examples thereof include a suspension form in which powder is diluted or suspended with a buffer solution such as water or phosphate buffered physiological saline (PBS).

For example, a typical pH range for formulating a pharmaceutical preparation for injection is about 2 to about 12. The pH of a part of the pharmaceutical composition according to the present embodiment may be within a range that is closer to the physiological pH. For example, in a specific embodiment, the pharmaceutical composition described in the present specification is formulated to exhibit a pH selected from a range of 4 to 10 or a range of 5 to 9.

### <<Method of producing pharmaceutical composition>>

The pharmaceutical composition according to the present embodiment can be produced by appropriately mixing the hyaluronic acid derivative with the active ingredient, but is preferably produced by the method described below.

That is, the method of producing a pharmaceutical composition according to the present embodiment is a method of producing a pharmaceutical composition containing a hyaluronic acid derivative and an active ingredient, and includes the following steps:
a preparation step of dispersing the active ingredient in water for injection or water containing a surfactant or a polymer to prepare a dispersion liquid containing the active ingredient, and a mixing step of optionally selecting a mixing ratio of the dispersion liquid and the water phase containing the hyaluronic acid derivative and mixing the dispersion liquid and the water phase.

**In** the related art, it is difficult to maintain the particle size of the hyaluronic acid derivative, for example, the interaction between the steryl groups is inhibited by the oil phase, and the particles are dispersed or the particles are partially aggregated, depending on the ratio of the oil phase in which the active ingredient is dissolved in the organic solvent and the water phase containing the hyaluronic acid derivative. Therefore, in some cases, the pharmaceutical composition contains only a complex of a hyaluronic acid derivative containing a relatively small-sized hyaluronic acid derivative in which particles are dispersed or a hyaluronic acid derivative in which particles are partially aggregated, and an active ingredient. In the method of producing a pharmaceutical composition according to the present embodiment, since the oil phase may not be used, it is possible to prepare a formulation in which the association of the hyaluronic acid derivative is maintained. This is excellent as an in situ gelation sustained-release base material because such a preparation leads to stable gelation and precipitation in a case of being administered in vivo.

Next, each step of the method of producing a pharmaceutical composition according to the present embodiment will be described in detail below.

### [Fine particle preparation step of active ingredient]

The method of producing the active ingredient can be adjusted according to a known method of purifying microparticles or nanoparticles. In the present embodiment, the micro or nano pulverization can be performed according to a known pulverization technology.

In the present embodiment, the micro or nano pulverization can be performed using one or a plurality of known wet pulverization technologies, supercritical or compressed fluid technologies, high temperature or high pressure homogenization, emulsification technologies, evaporation precipitation, anti-solvent precipitation, micro-precipitation, low temperature technologies, complexation technologies, ultrasonic treatment technologies, or solid dispersion technologies. Spray drying, freeze drying, and filtration separation can be used after the treatment in order to isolate the nanoparticles generated from the aqueous or solvent dispersion technology.

In the present embodiment, a method of obtaining microparticles or nanoparticles of the active ingredient may be a method of adding the active ingredient to a dispersion medium to obtain a coarse dispersion liquid in which the active ingredient is coarsely dispersed, and obtaining a dispersion liquid of microparticles or nanoparticles of the active ingredient by wet pulverization, or a method of adding the active ingredient to a dispersion medium after dry pulverization of the active ingredient in advance to obtain a dispersion liquid of microparticles or nanoparticles of the active ingredient.

It is preferable that the microparticles or nanoparticles of the active ingredient in the dispersion liquid are nanoparticles of the active ingredient obtained by wet pulverization.

The device used for the wet pulverization is not particularly limited, and examples thereof include a high-pressure homogenizer, a roll mill having a plurality of rolls, a colloid mill, a cone mill, a ball mill, and a bead mill.

The bead mill may be any of a batch type device, a circulation type device, and a continuous type device, or may be a combination thereof. The batch type device is a device that performs pulverization by putting the total amount of a liquid to be treated together with a medium for pulverization in a container for a bead mill. The circulation type device is a device that performs a treatment by circulating a liquid to be treated between a tank and a container for a bead mill. The continuous type device is a device in which a liquid to be treated continuously passes through a plurality of containers for a bead mill.

The diameter of the beads used in the bead mill is preferably 0.03 mm to 5 mm, more preferably 0.1 mm to 3 mm, still more preferably 0.1 mm to 1 mm, particularly preferably 0.1 mm to 0.5 mm, and even still more preferably 0.1 mm to 0.3 mm.

In a case where the diameter of the beads used in the bead mill is in the above-described ranges, the dispersion liquid after the wet pulverization treatment and the beads are easily carried out, and the active ingredient can be efficiently miniaturized.

Examples of the kind of beads include glass beads, low-alkali glass beads, non-alkali glass beads, zirconia-silica-based ceramic beads, yttrium-stabilized zirconia beads, silicon nitride beads, alumina beads, high-purity alumina beads, and titania beads. From the viewpoint of the record of use in the production of pharmaceutical products, yttrium-stabilized zirconia beads are preferable.

The yttrium-stabilized zirconia beads may also be simply referred to as zirconia beads.

In a case of preparing a dispersion liquid of microparticles or nanoparticles of the active ingredient, one or more selected from the group consisting of a surfactant, a polymer, and sugar (including sugar alcohol) may be added to a dispersion medium, and the active ingredient may be added to the solution or suspension, or the active ingredient may be added to the dispersion medium, and then one or more selected from the group consisting of a surfactant, a polymer, and sugar (including sugar alcohol) may be added thereto.

In a case where the dispersion liquid of the microparticles or nanoparticles of the active ingredient contains any one or both of a surfactant and a polymer, the mass ratio (active ingredient/surfactant/polymer) of the active ingredient to any one or both of the surfactant and the polymer in the dispersion liquid is preferably 1/1/1 to 1/0.01/0.01 and more preferably 1/0.75/0.75 to 1/0.1/0.1.

The active ingredient may be prepared as co-crystals of microparticles or nanoparticles by wet pulverization or the like.

In the present embodiment, the active ingredient may be suspended in a suspension medium including one or a plurality of kinds of carriers, and any one or both of an excipient and a diluent.

The microparticles are preferable from the viewpoint of ease of production. In addition, in a case where it is preferable that the particles are not phagocytized by phagocytes or the like in vivo, the microparticles are preferable. The dissolution rate can be decreased since the surface area is small from the viewpoint of the dissolution rate. In a case where the solubility of the compound is high and the long-term sustained release is difficult, the sustained-release period may be extended by using the microparticles.

The nanoparticles are preferable from the viewpoints of dispersion stability and ease of administration. Generally, nanoparticles having a small size and high dispersibility are easier to be administered than microparticles. The dissolution rate can be increased since the surface area is large from the viewpoint of the dissolution rate. In particular, in consideration of the purpose of increasing the dissolution rate of a poorly water-soluble compound, nanoparticles may be preferable.

### [Preparation step of aqueous carrier]

The aqueous carrier of the present embodiment contains a hyaluronic acid derivative. In this case, the hyaluronic acid derivative is used by being dissolved in a pharmaceutically acceptable medium at any concentration. For example, the above-described buffer solution such as water for injection or a phosphate buffer solution is preferable. The buffer is not particularly limited as long as it is a compound having a buffering capacity in a pH range of 4 to 10 of the composition. Examples of the buffer include acetates such as sodium acetate, phosphates such as sodium dihydrogen phosphate, disodium hydrogen phosphate, potassium dihydrogen phosphate, and dipotassium hydrogen phosphate, amino acid salts such as ε-aminocaproic acid and sodium glutamate, boric acid and salts thereof, and mixtures thereof. Among these, water for injection, a phosphate buffer solution, water for injection containing sucrose, or a phosphate buffer solution containing sucrose is particularly preferable.

The aqueous carrier of the present embodiment may contain a pH adjuster, and examples of the pH adjuster include hydrochloric acid, citric acid, phosphoric acid, acetic acid, tartaric acid, sodium hydroxide, potassium hydroxide, sodium carbonate, and sodium hydrogen carbonate.

An acid such as an organic acid or an inorganic acid can also be blended as necessary.

In addition, these pH adjusters may be used alone or a combination of two or more kinds thereof may be used.

The pH of the aqueous carrier of the present embodiment is not particularly limited as long as the pH thereof is in a range acceptable as a drug, but is, for example, in a range of 4.0 to 9.0, preferably 4.0 to 8.8, and more preferably 6.5 to 8.8.

The aqueous carrier of the present embodiment may contain a chelating agent, and examples of the chelating agent include disodium ethylenediaminetetraacetate, trisodium ethylenediaminetetraacetate, tetrasodium ethylenediaminetetraacetate, diethylenetriaminepentaacetate, and a mixture thereof.

The aqueous carrier of the present embodiment may contain an isotonic agent, and examples thereof include sodium chloride, glycerol, glucose, mannitol, sorbitol, and sucrose, which are commonly used. The isotonic agent may be added after the active ingredient is suspended.

Examples of other isotonic agents also include dextrose, lactose, mannitol, and a calcium or magnesium compound, such as CaCl₂.

Japanese Patent No. 4758893 describes that the antibacterial and preservative effect is improved by blending glycerol having a concentration (2% to 2.5% (v/v)) that substantially achieves isotonicity. Therefore, not only the function as an isotonic agent but also antibacterial and preservative effects are expected. The content of glycerol is, for example, 0.01% to 10% (w/v), preferably 0.05% to 5% (w/v), more preferably 0.1% to 3.0% (w/v), still more preferably 0.3% to 3.0% (w/v), and particularly preferably 0.3% to 2.5% (w/v).

A base can be blended into the aqueous carrier of the present embodiment as necessary. The base is not particularly limited as long as the base is allowed as a pharmaceutical product, and examples thereof include sodium hydroxide, potassium hydroxide, monoethanolamine, diethanolamine, triethanolamine, trometamol, and meglumine.

The aqueous carrier of the present embodiment can be further blended with one or two or more thickeners as necessary, and the thickeners are not particularly limited as long as the thickeners are acceptable as a drug.

Examples of the thickener of the aqueous carrier of the present invention include cellulose-based polymers (such as methyl cellulose, ethyl cellulose, hydroxyethyl cellulose, and hydroxypropyl methyl cellulose), vinyl-based polymers (such as polyvinylpyrrolidone and polyvinyl alcohol), saccharides (such as hyaluronic acid and mucopolysaccharides such as salts thereof, and polysaccharides such as gellan gum, sodium alginate, dextran, and cyclodextrin), and oxyalkylene-based polymers (such as a polyoxyethylene polyoxypropylene block copolymer). The molecular weight of the thickener in the present invention can be selected from, for example, a range of a number average molecular weight of about 0.5 × 10⁴ to 100 × 10⁴.

One or two or more kinds of preservatives can be further blended into the aqueous carrier of the present embodiment as necessary, and the preservatives are not particularly limited as long as the preservatives are acceptable as a drug.

As the preservative of the aqueous carrier in the present invention, for example, benzalkonium chloride, benzethonium chloride, chlorhexidine gluconate, paraoxybenzoic acid esters such as methyl paraoxybenzoate and ethyl paraoxybenzoate, benzyl alcohol, phenethyl alcohol, sorbic acid or a salt thereof, thimerosal, chlorobutanol, and the like are used.

### [Mixing step]

In the mixing step, the dispersion liquid of the fine particles obtained in the preparation step and the water phase containing the hyaluronic acid derivative may be mixed at any mixing ratio. For example, a mixing ratio between the dispersion liquid of the fine particles and the water phase containing the hyaluronic acid derivative is preferably 0.01:100 (v/v) to 100: 100 (v/v). As the proportion of the water phase containing the hyaluronic acid derivative increases, a concentration gradient of the active ingredient in the aqueous carrier is unlikely to occur, and the active ingredient is likely to be uniformly dispersed. The dose of the active ingredient can be increased as the ratio of the dispersion liquid of the fine particles increases.

In the mixing step, the concentration of the hyaluronic acid derivative dissolved in the water phase is not particularly limited, but the concentration of the hyaluronic acid derivative is preferably 0.01 parts by mass or greater and 10 parts by mass or less, more preferably 0.1 parts by mass or greater and 10 parts by mass or less, and still more preferably 0.5 parts by mass or greater and 5 parts by mass or less with respect to 100 parts by mass of the water phase.

After the mixing step, the concentration of the hyaluronic acid derivative in the pharmaceutical composition of the present embodiment is not particularly limited, but is preferably 0.01 parts by mass or greater and 10 parts by mass or less, more preferably 0.05 parts by mass or greater and 7.5 parts by mass or less, and still more preferably 0.3 parts by mass or greater and 5 parts by mass or less with respect to 100 parts by mass of the pharmaceutical composition.

In the mixing step, the temperature is not particularly limited. From the viewpoint of reducing the viscosity of the aqueous carrier, the temperature is preferably 50°C or higher, and from the viewpoint of the stability of the active ingredient, the temperature is preferably 50°C or lower, more preferably 10°C or higher and 55°C or lower, and still more preferably 15°C or higher and 40°C or lower. Alternatively, the optimum temperature can be appropriately set depending on the structure and the characteristics of the active ingredient. In addition, for example, a step of adjusting the temperature of the dispersion liquid of fine particles to 15°C to 25°C and adjusting the temperature of the aqueous carrier containing the hyaluronic acid derivative to 40°C to 50°C, and mixing two or more solutions having different temperatures may be provided.

In the mixing step, the step is not limited only to the batch method. The dispersion liquid of the active ingredient and the aqueous carrier may be mixed by a flow method.

The material and the shape of the reactor in the flow method are not particularly limited as long as a material and a shape applicable to the production method according to of the present invention are selected, and for example, the inner diameters of the pipes through which each of the dispersion liquid and the aqueous carrier pass may be different from each other.

The material and the shape of the tube in the flow method are not particularly limited as long as a material and a shape applicable to the method of producing a pharmaceutical composition of the present embodiment are selected, and examples thereof include a tube made of Teflon (registered trademark), a stainless-steel pipe, a glass pipe, and a plastic pipe.

In the mixing step, the time is not particularly limited, but for example, the time can be set to 30 minutes or longer and 90 hours or shorter, or 1 hour or longer and 80 hours or shorter.

### [Drying step]

After the mixing step, a drying step of drying the obtained solution containing the active ingredient and the hyaluronic acid derivative to obtain a dried product may be performed. Examples of the drying method include the same method as the method described in the section "method of producing hyaluronic acid derivative" above. Examples thereof include freeze drying, spray drying, a vacuum dryer, and supercritical drying.

### <Administration method>

The target to which the pharmaceutical composition of the present embodiment is administered includes animals (monkeys, marmosets, mice, rats, cows, horses, cats, dogs, pigs, sheep, goats, rabbits, and the like) classified as mammals including humans.

The route of administration of the pharmaceutical composition of the present embodiment is not particularly limited, and can be appropriately used for a known route of administration at present depending on the intended use, the position of the tissue to be treated, and the like.

Examples of the administration route include subcutaneous administration, intramuscular administration, intravenous administration, intraarterial administration, intrathecal administration, intracerebral administration, intraarticular administration, intraperitoneal administration, intravaginal administration, intravesicular administration, intrarectal administration, intravitreal administration, periorbital administration, intracutaneous administration, intraperitoneal administration, intranasal administration, intrabronchial administration, intrapulmonary administration, transdermal administration, sublingual administration, oral administration, buccal administration, and instillation administration. Among these, subcutaneous administration, intramuscular administration, intravitreal administration, intrathecal administration, and intraarticular administration are preferable.

In addition, the pharmaceutical composition may be used as a powder, a cream, an ointment, or an eye drop for local administration.

Examples of the administration by injection include subcutaneous injection, intramuscular injection, intravenous injection, intra-arterial injection, intrathecal injection, intraarticular injection, intraperitoneal injection, intravitreal injection, periorbital injection, intradermal injection, and intratumoral injection.

It is preferable that the pharmaceutical composition is administered subcutaneously, intramuscularly, intraspinally, intraarticularly, intracerebrally, intravitreally, or to an ocular surface, a nose, a lung, or a mouth as an administration site. It is more preferable that the pharmaceutical composition is administered subcutaneously, intramuscularly, intraspinally, intraarticularly, intravitreally, or to an ocular surface as an administration site.

It is preferable that the administration method is injection, eye drop, or application.

In the eye drop, in a case where the hyaluronic acid derivative is used, it is possible to reduce the initial burst of the drug, to suppress aggregation that may occur between drugs during the storage of the preparation and after the administration of the drug, to reduce the variation in drug efficacy, to allow the drug to be sustained released for a longer period of time by precipitating under physiological conditions and adhering to the corneal epithelium, and to reduce the number of times of eye drop. Further, it is also considered that the uptake of a drug by corneal epithelial cells and the uptake of a drug by conjunctival epithelial cells are promoted. In addition, it is possible to provide a preparation having high moisturizing properties, viscosity, and biocompatibility derived from hyaluronic acid, which can alleviate inflammation, pain, stress, damage, and the like during contact with the preparation, and has high safety. As described above, a pharmaceutical composition having an excellent effect of stabilizing a tear film layer, an effect of treating corneal epithelial injury, an effect of treating meibomian gland dysfunction, and an effect of suppressing pain is provided by efficiently bringing out the effect of the drug.

In the intra-articular administration, the compatibility with hyaluronic acid present in the synovial fluid is satisfactory, and the initial burst of the drug can be effectively suppressed by precipitating the hyaluronic acid derivative. Further, the precipitate of the hyaluronic acid derivative that retains the drug can be retained in the knee joint for a longer period of time by administering the hyaluronic acid derivative together with a high-molecular-weight hyaluronic acid (Alzheimer's disease, Suvenyl, or the like) which is an existing pharmaceutical product used for OA treatment or the like.

In the pharmaceutical composition of the present embodiment, in a case of being administered parenterally, the dose can be appropriately selected in consideration of the kind of the administration target (including the age, the gender, and the like), but usually, for example, in a case of a human (assuming a body weight of 60 kg), the amount of the active ingredient per dose can be set to 0.01 µg or greater and 100 mg or less, 0.1 µg or greater and 50 mg or less, or 1 µg or greater and 25 mg or less.

The number of times of administration may be single administration of the above-described dose or once or twice or more of a plurality of times of administration of the above-described dose, for example, every week, every 2 weeks, every 3 weeks, every 4 weeks, every month, every 2 months, every 3 months, or every half a year. Alternatively, two or more sites may be administered in one administration.

### <<Other embodiments>>

In one embodiment, the present invention provides a method of preventing or treating one or more diseases selected from the group consisting of cancer, infectious disease, immune disease, inflammatory laxity, allergic disease, skin disease, hypertension, diabetes, nervous disease, hereditary disease, cardiovascular disease, cerebrovascular disease, respiratory disease, eye disease, ear disease, bone joint disease, and pain disease, the method including administering an effective amount of the pharmaceutical composition to a patient or a patient animal.

In addition, the term "effective amount" as used herein includes an amount that is effective for prevention or treatment, that is, an amount suitable for preventing or treating the above-described disease.

In one embodiment, the present invention provides a composition for preventing or treating one or more diseases selected from the group consisting of cancer, infectious disease, immune disease, inflammatory relaxation, allergic disease, skin disease, hypertension, diabetes, nervous disease, hereditary disease, cardiovascular disease, cerebrovascular disease, respiratory disease, eye disease, ear disease, bone joint disease, and pain disease, and the composition contains the active ingredient-hyaluronic acid derivative complex.

In one embodiment, the present invention provides the use of the above-described active ingredient and hyaluronic acid derivative mixture for producing a pharmaceutical composition.

In one embodiment, the present invention provides a method for long-term sustained release of an active ingredient in vivo or in vitro, the method including administering a composition containing the active ingredient and the hyaluronic acid derivative.

One aspect of the present invention relates to a pharmaceutical composition containing an active ingredient suspended in an aqueous carrier containing a base material that is precipitated or salted out in vivo or under a physiological condition, in which the active ingredient is a fine particle having a molecular weight of 200 g/mol or greater and 5,000 g/mol or less.

The expression "base material that is precipitated or salted out in vivo or under a physiological condition" is, for example, a hyaluronic acid derivative, poloxamer, unmodified chitosan, a chitosan derivative, or a peptide analog.

In one aspect of the present invention, the base material that is precipitated or salted out in vivo or under a physiological condition is preferably a hyaluronic acid derivative and preferably a hyaluronic acid derivative pharmaceutical composition containing an active ingredient suspended in an aqueous carrier.

In a case where the hyaluronic acid derivative is used as a base material, inflammation is unlikely to occur at the administration site, and biocompatibility is excellent. Further, the carboxylic acid of the hyaluronic acid derivative and the divalent salt in vivo are ionically crosslinked, or the modified steryl groups are highly crosslinked with each other, whereby the drug can be more stably retained than in other base materials, and initial burst suppression and a long-term sustained-release effect can be obtained.

In one aspect of the present invention, whether or not the base material is precipitated or salted out in vivo or under a physiological condition can be confirmed by the following method.

First, the base material dissolved to a desired concentration is added to a phosphate buffer solution or a phosphate buffer solution containing 0.8% BSA. Thereafter, the mixture is incubated at 37°C and centrifuged, and in a case where the solid-liquid separation can be carried out, it is determined that the base material is precipitated or salted out with physiological saline. The incubation time can be optionally selected.

The concentration of the phosphate buffer solution is, for example, 1 × PBS (about 10 mM PB, 150 mM NaCl) in terms of the final concentration. The pH is near neutral and is usually in a range of 7.1 to 7.7.

Centrifugal conditions in the case of centrifugation can be appropriately changed, and an example thereof is 9800 g.

In one aspect of the present invention, the proportion of the precipitated and salt-out chitosan or peptide analog under physiological conditions can be quantified by adding 150 µL of 4 × PBS (final concentration: 1 × PBS) to 450 µL of the chitosan or peptide analog dissolved at a desired concentration, incubating the mixture under the above-described conditions, and quantifying the supernatant of the hyaluronic acid derivative sedimented due to centrifugation by size exclusion chromatography (SEC) or reverse phase chromatography (HPLC). The quantification may be carried out by either SEC or HPLC, and any one of these is appropriately selected.

In one aspect of the present invention, the proportion of the precipitated and salt-out chitosan or peptide analog under physiological conditions can be quantified by adding 150 µL of 4 × PBS containing 3.2% BSA (final concentration: 1 × PBS containing 0.8% BSA) to 450 µL of the chitosan or peptide analog dissolved at a desired concentration, incubating the mixture under the above-described conditions, and quantifying the supernatant of the hyaluronic acid derivative sedimented due to centrifugation by size exclusion chromatography (SEC) or reverse phase chromatography (HPLC). The quantification may be carried out by either SEC or HPLC, and any one of these is appropriately selected.

In one aspect of the present invention, the precipitated and salt-out hyaluronic acid derivative under the physiological salt conditions can be confirmed by actually administering the hyaluronic acid derivative in vivo to confirm the precipitation or by administering the hyaluronic acid derivative to a collected tissue to confirm the precipitation.

The proportion of the precipitate is preferably 10% to 100%, preferably 40% or greater, 50% or greater, and 60% or greater, more preferably 70% or greater, still more preferably 80% or greater, and most preferably 90% or greater with respect to the total amount of the dissolved hyaluronic acid derivative.

The base material that is precipitated or salted out in vivo or under a physiological condition is gelated after being administered into the body. Due to this action, the fine particles of the active ingredient contained in the pharmaceutical composition are less likely to be aggregated or diffused. As a result, a pharmaceutical composition in which the concentration of the active ingredient is maintained in vivo for a long period of time without variation and the initial release amount abnormality (initial burst) that is reduced is likely to be provided.

### Examples

Hereinafter, the present invention will be described in detail with reference to examples, but the present invention is not intended to be limited to the examples.

### <Example 1>

A hyaluronic acid derivative pharmaceutical composition was produced by the following method.

### [Preparation of cyclosporin A suspension]

720 mg of cyclosporin A ((manufactured by Tokyo Chemical Industry Co., Ltd., product number: C2408)) was weighed in a 30 mL glass vial, and 14.4 mL of 0.01% polysorbate 80 was added thereto. Further, 10 g of 0.2 mm ZrO2 beads (manufactured by Nikka Co., Ltd.) were added thereto, and 14.4 mL of 1% polysorbate 80 was added thereto. The rotor was placed therein, and the mixture was stirred with a magnetic stirrer for 11 days and finely pulverized. The suspension of cyclosporin A was taken out with a syringe, placed in a 15 mL conical tube, centrifuged at 10,000 g for 60 minutes, and the supernatant was discarded. Further, 10 times the amount of water for injection was added thereto, and the mixture was centrifuged at 10,000 g for 60 minutes, and the supernatant was discarded, thereby obtaining a suspension of cyclosporin. The cyclosporin suspension was diluted 200 times with a 0.05% polysorbate 80 aqueous solution using a DLS device (manufactured by Otsuka Electronics Co., Ltd., ELSZ2000) for nanosizing the cyclosporin, and the particle diameter of the cyclosporin was measured. As a result, the average particle diameter thereof was 171 nm and the polydispersity index thereof was 0.12. As a result of quantifying the concentration of cyclosporin under the following conditions, the concentration thereof was 117.7 mg/mL.

### (Conditions for reverse phase chromatography analysis)

HPLC device: JASCO HPLC-EXTREMA

### [HPLC quantification conditions: reverse phase chromatography analysis conditions]

Column: Inert Sustain C18, particle diameter 5 µm × inner diameter 4.6 mm × length 150 mm
Column temperature: 40°C
Mobile phase: eluent A, 0.1% TFA/acetonitrile
Eluent B: 0.1% TFA/water
Ratio between mobile phases: eluent A/eluent B = 9/1
Flow rate: 1 mL/min
Injection volume: 30 µL
Detector: UV (210 nm)

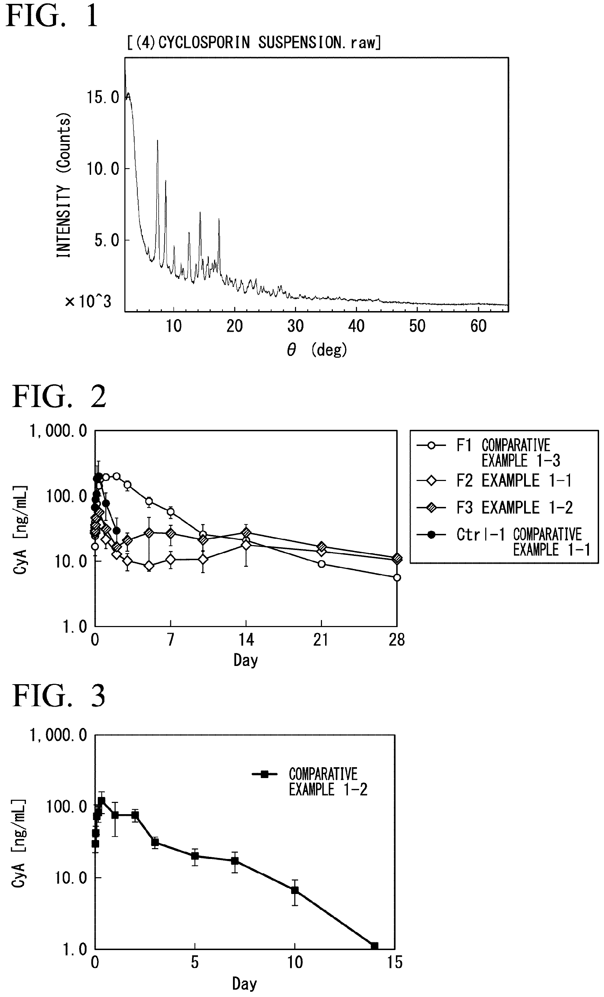

The suspension of cyclosporin obtained above was vacuum-dried, and the obtained powder was placed on a non-reflective sample table and measured under the following conditions. The obtained X-ray powder diffraction peak is shown in FIG. 1. It can be seen that the crystalline property is exhibited.

### (Measurement conditions for XRD)

Measuring device: Rigaku Ultima-IV
X-ray source: Cu-Kα
Excitation voltage: voltage of 40 kV, current of 40 mA
Optical system: converging optical system
Cu-Kβ ray filter: Ni foil
Absorber: none
Detector: Dtex (high-sensitivity detector)
Measuring method: θ/2θ method
Slit: DS = 1°, SS = open, RS = open, vertical slit = 10 mm
2θ/θ scan: 2θ = 2° to 65° (0.02°/step, 10°/min)

The cyclosporin obtained above was a nanoparticle having a molecular weight of 1202.6 g/mol and a particle diameter of 171 nm.

### [Preparation of hyaluronic acid nanogel]

A hyaluronic acid nanogel (weight-average molecular weight: 35 kDa, introduction rate of cholesteryl group: 19%) in which a cholesteryl group was introduced was synthesized as described in Examples 1 and 2 of PCT International Publication No. WO2010/053140. The synthesized hyaluronic acid nanogel may also be referred to as "HA derivative".

A phosphate buffer solution was added to the HA derivative dissolved at a concentration of 6 mg/mL. Thereafter, the mixture was incubated at 37°C and centrifuged, and thus solid-liquid separation could be carried out. Therefore, it was determined that the HA derivative was precipitated or salted out with physiological saline.

A 12 mg/mL aqueous solution of the HA derivative (35k19) synthesized in [Preparation of hyaluronic acid nanogel] was mixed with the cyclosporin A suspension prepared in [Preparation of cyclosporin A suspension], and a 10% sucrose solution was further added thereto. In this manner, the hyaluronic acid derivative pharmaceutical compositions of Examples 1-1 and 1-2 were produced. The prepared samples are listed in Table 1. In the following table, "CyA concentration" denotes the concentration of the cyclosporin A. The final sucrose concentration was 8.7%.

**[Table 1]**

| | CyA concentration | 35k19 concentration |
|---|---|---|
| Example 1-1 | Suspension | 10 mg/mL |
| | 10 mg/mL | |
| Example 1-2 | Suspension | 5 mg/mL |
| | 10 mg/mL | |

### <Comparative Example 1-1>

The cyclosporin A powder was dissolved in a 10% α-cyclodextrin/10% sucrose solution at a proportion of 0.5 mg/mL in Comparative Example 1-1.

### <Comparative Example 1-2>

An ethanol solution of 200 mg/mL of cyclosporin A was mixed with an aqueous solution of 12 mg/mL of the HA derivative (35k19) synthesized in [Preparation of hyaluronic acid nanogel] described above, and the mixture was stirred overnight. Sucrose was added in the form of powder to prepare a 10% sucrose solution. The final cyclosporin concentration was 1.0 mg/mL, and the 35k19 concentration was 9 mg/mL.

### <Comparative Example 1-3>

A 1% polysorbate 80 aqueous solution and a 10% sucrose solution were mixed with the cyclosporin A suspension prepared in [Preparation of cyclosporin A Suspension] described above. The final cyclosporin concentration was 10 mg/mL, the polysorbate 80 concentration was 0.05%, and the sucrose concentration was 8.7%.

### <Pharmacokinetic test>

Each of the samples of Example 1-1, Example 1-2, and Comparative Example 1-1 to 1-3 prepared in Example 1 was subcutaneously administered to a normal rat (SD, 6 weeks oil, male) using a syringe with a 25 G needle at the dose listed in Table 2.

**[Table 2]**

| | Form and concentration of CyA | 35k19 concentration | Dose of CyA (mg/kg) |
|---|---|---|---|
| Comparative Example 1-1 | Solution | 0 mg/mL | 2.2 mg/kg |
| | 0.5 mg/mL | | |
| Comparative Example 1-2 | Solution | 9 mg/mL | 5.0 mg/kg |
| | 0.8 mg/mL | | |
| Comparative Example 1-3 | Suspension | 0 mg/mL | 25 mg/kg |
| | 10 mg/mL | | |
| Example 1-1 | Suspension | 10 mg/mL | 25 mg/kg |
| | 10 mg/mL | | |
| Example 1-2 | Suspension | 5 mg/mL | 25 mg/kg |
| | 10 mg/mL | | |

After the administration, blood was collected from the jugular vein with a syringe treated with heparin over time, and EDTA-K₂ was added as a protease inhibitor.

The obtained blood was separated into plasma and measured by LC-MS/MS. FIGS. 2 and 3 show changes in the blood plasma concentration of cyclosporin at the time of the administrating various cyclosporin suspension preparations.

As shown in FIG. 2, in Comparative Examples 1-1 and 1-3, the initial release amount abnormality was confirmed. On the contrary, in Example 1-1 and Example 1-2, no initial release amount abnormality was confirmed.

Further, as shown in FIG. 2, it was confirmed that in Example 1-1 and Example 1-2, the blood concentration of 10 ng/mL or greater was maintained even 28 days after the administration, and the long-term sustained-release properties were exhibited.

Meanwhile, in Comparative Examples 1 to 3, the blood concentration was reduced to less than 7 ng/mL 28 days after the administration, and the long-term sustained-release effect could not be confirmed.

As shown in this result, it was shown that even in a case where the active ingredient is suspended, the initial release amount abnormality is likely to occur and the long-term sustained release is not exhibited in a case where the active ingredient is not suspended in the aqueous carrier containing the hyaluronic acid derivative because the fine particles of the active ingredient are diffused in vivo.

In Comparative Example 1-1 in which the active ingredient was not suspended and the aqueous carrier did not contain a hyaluronic acid derivative, cyclosporin was not detected in the blood after 7 days. That is, the sustained-release effect was hardly recognized. As shown in this result, it was shown that since α-cyclodextrin is gelated and is not precipitated at a physiological saline concentration after subcutaneous administration, gelation and precipitation in situ are required for long-term sustained release, as in the case of a hyaluronic acid derivative.

In addition, as shown in FIG. 3, in the pharmaceutical composition of Comparative Example 1-2 in which the active ingredient formed a complex with the hyaluronic acid derivative and the active ingredient was not suspended, the initial release amount abnormality was confirmed, the blood concentration was reduced to less than 7 ng/mL 10 days after the administration, and thus the long-term sustained-release effect could not be confirmed.

As shown in this result, in a case where the active ingredient was not suspended in the aqueous carrier containing the hyaluronic acid derivative, the hyaluronic acid derivative was gelated in situ at a physiological saline concentration, but the long-term sustained-release effect could not be obtained.

In the present specification, the sustained-release properties are evaluated by the mean residual time (MRT), and more specifically, in a case where Expression (A) is satisfied, the sustained-release properties are evaluated that "the concentration of the active ingredient can be maintained in vivo for a long period of time". (MRT (time) of hyaluronic acid derivative pharmaceutical composition)/(MRT (time) of active ingredient alone) ≥ 1.2

As a result of evaluating the sustained-release properties by Expression (A), it was found that the sustained-release properties of Example 1-1 were 3.8 as compared with Comparative Example 1-3, and the sustained-release properties of Example 1-2 were 2.8 as compared with Comparative Example 1-3, which were confirmed to have sustained-release properties.

In the present specification, the initial release amount abnormality (initial burst) is evaluated by the maximum blood concentration Cmax, and more specifically, in a case where Expression (B) is satisfied, it is evaluated that "the initial release amount abnormality (initial burst) can be reduced". {(Maximum blood concentration Cmax [ng/mL] of hyaluronic acid derivative pharmaceutical composition) ÷ (dose [mg/kg] of active ingredient)} ÷ {(Cmax [ng/mL] of active ingredient alone) ÷ (dose [mg/kg] of active ingredient)} ≤ 0.8

In a case of evaluating the initial burst by Expression (B), it was found that the initial burst of Example 1-1 was 0.21 as compared with Comparative Example 1-3, and initial burst of Example 1-2 was 0.30 as compared with Comparative Example 1-3, which were confirmed that the initial release amount abnormality could be reduced.

As described above, the following pharmacokinetic parameters were analyzed by noncompartmental analysis using WinNonlin Ver. 8.3 (manufactured by Pharsight Corporation), and the values thereof are listed in Table 3.
· Average retention time (MRT), unit: hour
· Half-life (T1/2), unit: hour
· Maximum blood concentration (Cmax), unit: ng/mL

**[Table 3]**

| | Dose of CyA (mg/kg) | T1/2 (h) | MRT (h) | Cmax (ng/mL) |
|---|---|---|---|---|
| Comparative Example 1-1 | 2.2 mg/kg | 20.1 | 26.9 | 211 |
| Comparative Example 1-2 | 5.0 mg/kg | 50.6 | 81.1 | 121 |
| Comparative Example 1-3 | 25 mg/kg | 184 | 183 | 200 |
| Example 1-1 | 25 mg/kg | 391 | 690 | 41.5 |
| Example 1-2 | 25 mg/kg | 311 | 518 | 59 |

### <Experimental Example 1: evaluation of precipitation>

In a case where Example 1-1 and Example 1-2 prepared in Example 1 were added dropwise to a 0.8% BSA/PBS solution using a syringe with a 30G needle, a precipitate into which cyclosporin in a solid state was mixed was formed.

Meanwhile, in a case where the comparative sample 1-3 prepared in Comparative Example 1-3 was added dropwise to the above-described solution, no precipitate was formed.

### <Experimental Example 2: evaluation of precipitation>

The influence of the kind of the hyaluronic acid derivative was verified.

### [Preparation of hyaluronic acid nanogel]

A hyaluronic acid nanogel (weight-average molecular weight: 10 kDa, introduction rate of cholesteryl group: 30%) in which a cholesteryl group was introduced was synthesized as described in Examples 1 and 2 of PCT International Publication No. WO2010/053140.

A 30 mg/mL aqueous solution of the HA derivative (10k30) synthesized in [Preparation of hyaluronic acid nanogel] was mixed with the cyclosporin A suspension prepared in [Preparation of cyclosporin A suspension] of Example 1, and then adjusted with water for injection to have the concentration listed in Table 4. In this manner, a hyaluronic acid derivative pharmaceutical composition of Example 2-1 was produced. The prepared samples are listed in Table 4. In the following table, "CyA concentration" denotes the concentration of the cyclosporin A.

**[Table 4]**

| | CyA concentration | 10k30 concentration |
|---|---|---|
| Example 2-1 | Suspension | 20 mg/mL |
| | 10 mg/mL | |

In a case where Example 2-1 was added dropwise to a 0.8% BSA/PBS solution using a syringe having a 30G needle, a precipitate into which cyclosporin in a solid state was mixed was formed.

### <Experimental Example 3: evaluation of precipitation in vitreous body>

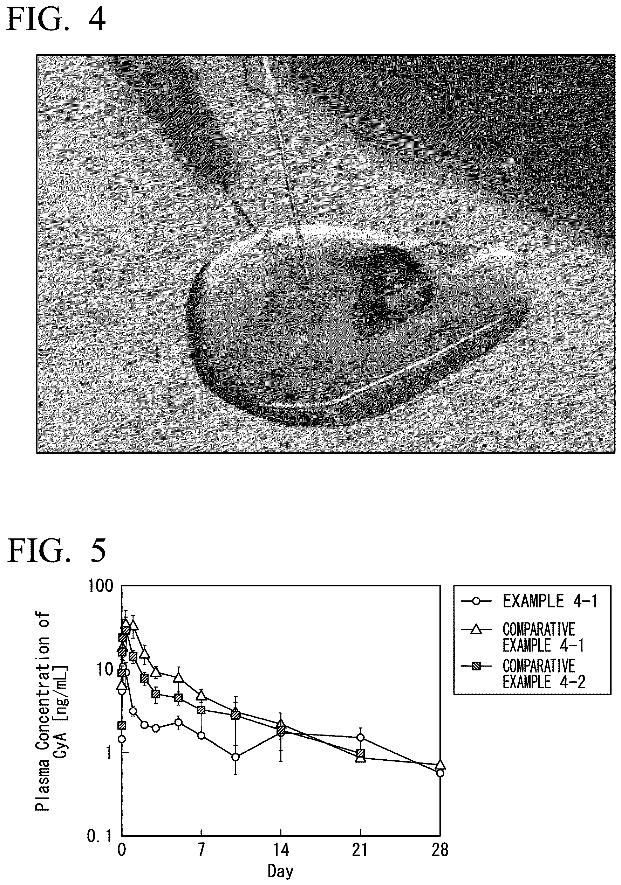

A vitreous body solution was extracted from a porcine eyeball (COSMO BIO Co., Ltd.). Example 1-1 prepared in Example 1 was administered to the vitreous body solution using a syringe. The results are shown in FIG. 4.

As shown in FIG. 4, the formation of a precipitate into which cyclosporin in a solid state was mixed could be confirmed.

### <Example 4: intravitreal administration>

A hyaluronic acid derivative pharmaceutical composition was produced by the following method.

### [Preparation of cyclosporin A suspension]

A suspension of cyclosporin was obtained by the same method as in Example 1. In a case where the measurement was performed using a DLS device (manufactured by Otsuka Electronics Co., Ltd., ELSZ2000), the average particle diameter was 171 nm and the polydispersity index was 0.12. As a result of quantifying the concentration of cyclosporin under the following conditions, the concentration thereof was 117.7 mg/mL.

### (Reverse phase chromatography analysis conditions)

HPLC device: JASCO HPLC-EXTREMA

### [HPLC quantification conditions: reverse phase chromatography analysis conditions]

Column: Inert Sustain C18, particle diameter 5 µm × inner diameter 4.6 mm × length 150 mm
Column temperature: 40°C
Mobile phase: eluent A, 0.1% TFA/acetonitrile
Eluent B: 0.1% TFA/water
Ratio between mobile phases: eluent A/eluent B = 9/1
Flow rate: 1 mL/min
Injection volume: 30 µL
Detector: UV (210 nm)

### [Preparation of hyaluronic acid nanogel]

A hyaluronic acid nanogel (weight-average molecular weight: 35 kDa, introduction rate of cholesteryl group: 19%) in which a cholesteryl group was introduced was synthesized as described in Examples 1 and 2 of PCT International Publication No. WO2010/053140.

12 mg/mL of a 10% sucrose-containing aqueous solution containing the HA derivative (35k19) synthesized in [Preparation of hyaluronic acid nanogel] was mixed with the cyclosporin A suspension prepared in [Preparation of cyclosporin A suspension], and a 10% sucrose solution was further added thereto. In this manner, a hyaluronic acid derivative pharmaceutical composition of Example 4-1 containing a CyA concentration of 40 mg/mL was produced. The final glucose concentration was 6.6%.

### <Comparative Example 4-1>

The cyclosporin A suspension prepared in [Preparation of cyclosporin A suspension] was mixed with a 0.05% polysorbate aqueous solution at a proportion set such that the CyA concentration was 40 mg/mL, and a 10% sucrose solution was further added thereto in Comparative Example 4-1. The final glucose concentration was 6.1%.

### <Comparative Example 4-2>

A powder of 1000 kDa hyaluronic acid (manufactured by Kikkoman Corporation, FCH-120) was weighed in a sterile vial and dissolved in a 10% sucrose aqueous solution at a concentration of 10 mg/mL. The adjusted 1000 kDa hyaluronic acid (manufactured by Kikkoman Corporation, FCH-120) aqueous solution, the cyclosporin A suspension prepared in [Preparation of cyclosporin A suspension] described above, and a 10% sucrose aqueous solution were mixed at a proportion set such that the CyA concentration was 40 mg/mL and the hyaluronic acid concentration was 6.6 mg/mL in Comparative Example 4-2. The final glucose concentration was 6.6%.

### <Pharmacokinetic test>

Each of the samples of Example 4-1 and Comparative Examples 4-1 and 4-2 prepared in Example 4 was administered into the vitreous body of a normal rat (SD, 6 weeks old, male) at a dose of 0.8 mg/head using a syringe having a 28G needle.

**[Table 5]**

| | Form and concentration of CyA | 35k19 concentration |
|---|---|---|
| Example 4-1 | Suspension | 5 mg/mL |
| | 40 mg/mL | |
| Comparative Example 4-1 | Suspension | 0 mg/mL |
| | 40 mg/mL | |
| Comparative Example 4-2 | Suspension | 0 mg/mL |
| | 40 mg/mL | |

After the administration, blood was collected from the jugular vein with a syringe treated with heparin over time, and EDTA-K₂ was added as a protease inhibitor.

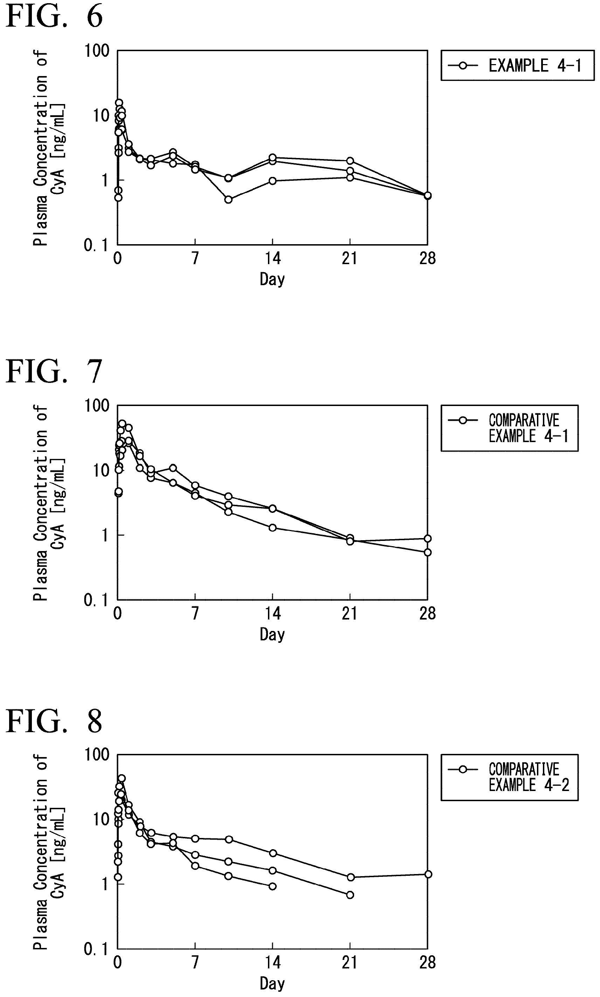

The obtained blood was separated into plasma and measured by LC-MS/MS. FIG. 5 shows a change in the blood plasma concentration of cyclosporin at the time of administering various cyclosporin suspension preparations. In addition, FIG. 6 shows a change in the blood plasma concentration of cyclosporin at the time of administering the preparation of Example 4-1, FIG. 7 shows a change in the blood plasma concentration of cyclosporin at the time of administering the preparation of Comparative Example 4-1, and FIG. 8 shows a change in the blood plasma concentration of cyclosporin at the time of administering the preparation of Comparative Example 4-2.

As shown in FIG. 5, in Comparative Example 4-1 and Comparative Example 4-2, the initial release amount abnormality was confirmed. On the contrary, in Example 4-1, the initial release amount abnormality was not confirmed.

Further, as shown in the results of FIGS. 6 to 8, in Comparative Example 4-1 and Comparative Example 4-2, CyA could not be sustained released for a long period of time (28 days) in all groups in a case of n = 3, whereas in Example 4-1, the variation was small and the long-term sustained release could be confirmed in all the administration groups in a case of n = 3.

As shown in this result, it was found that even in a case where the active ingredient is suspended after administration, the initial release amount cannot be suppressed with hyaluronic acid. It was found that even in a case of a highly viscous preparation such as hyaluronic acid, since the hyaluronic acid is not gelated or precipitated at a physiological saline concentration, gelation and precipitation in situ are required for long-term sustained release as in the case of a hyaluronic acid derivative.

Subsequently, the following pharmacokinetic parameters were analyzed by noncompartmental analysis using WinNonlin Ver. 8.3 (manufactured by Pharsight Corporation) by the same method as in Example 1, and the values thereof are listed in Table 6.
· Average retention time (MRT), unit: hour
· Half-life (T1/2), unit: hour
· Maximum blood concentration (Cmax), unit: ng/mL

**[Table 6]**

| | T1/2 (h) | MRT (h) | Cmax (ng/mL) |
|---|---|---|---|
| Example 4-1 | 178.7 | 343.8 | 10.7 |
| Comparative Example 4-1 | 162.5 | 198.3 | 29.1 |
| Comparative Example 4-2 | 140.9 | 158.5 | 36.4 |

As a result of evaluating the sustained-release properties by Expression (A), it was found that the sustained-release properties of Example 4-1 were 1.7 as compared with Comparative Example 4-1, and the sustained-release properties of Example 4-1 were 2.2 as compared with Comparative Example 4-2, which were confirmed to have sustained-release properties.

In a case where the initial burst was evaluated by Expression (B), it was found that the initial burst of Example 4-1 was 0.37 as compared with Comparative Example 4-1, and initial burst of Example 4-1 was 0.29 as compared with Comparative Example 4-2, which were confirmed that the initial release amount abnormality could be reduced.

### <Example 5: Intra-articular administration>

The same preparation as in Example 4 was used for the hyaluronic acid derivative pharmaceutical composition of Example 5-1, the polysorbate-containing pharmaceutical composition of Comparative Example 5-1, and the hyaluronic acid-containing pharmaceutical composition of Comparative Example 5-2.

### <Pharmacokinetic test>

Each of the samples of Example 5-1 and Comparative Examples 5-1 and 5-2 prepared in Example 4 was administered into the knee joint of a normal rat (SD, 6 weeks old, male) at a dose of 0.8 mg/head using a syringe having a 28 G needle.

**[Table 7]**

| | Form and concentration of CyA | 35k19 concentration |
|---|---|---|
| Example 5-1 | Suspension | 5 mg/mL |
| | 40 mg/mL | |
| Comparative Example 5-1 | Suspension | 0 mg/mL |
| | 40 mg/mL | |
| Comparative Example 5-2 | Suspension | 0 mg/mL |
| | 40 mg/mL | |

After the administration, blood was collected from the jugular vein with a syringe treated with heparin over time, and EDTA-K₂ was added as a protease inhibitor.

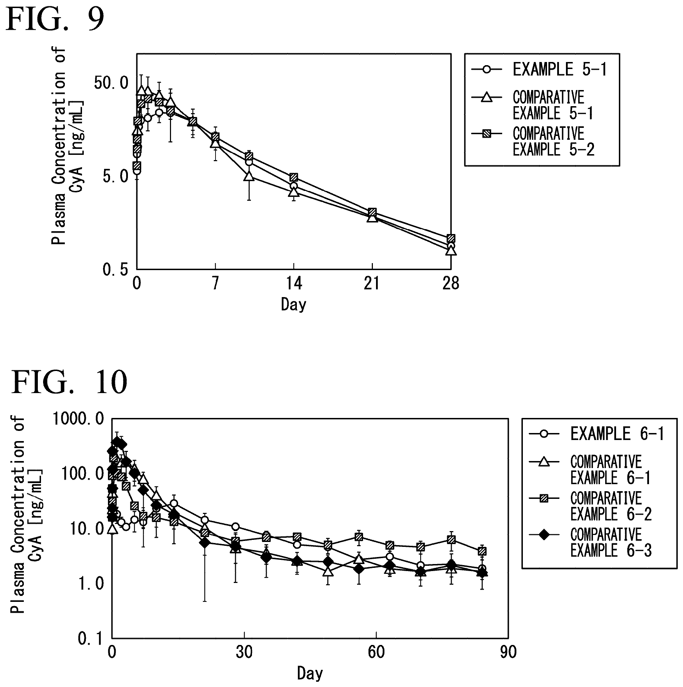

The obtained blood was separated into plasma and measured by LC-MS/MS. FIG. 9 shows a change in the blood plasma concentration of cyclosporin at the time of administering various cyclosporin suspension preparations.

As shown in FIG. 5, in Comparative Examples 5-1 and 5-2, the initial release amount abnormality was confirmed. On the contrary, in Example 5-1, the initial release amount abnormality was not confirmed.

Further, as shown in the results of FIGS. 6 to 8, in Comparative Example 4-1 and Comparative Example 4-2, CyA could not be sustained released for a long period of time (28 days) in all groups in a case of n = 3, whereas in Example 4-1, the variation was small and the long-term sustained release could be confirmed in all the administration groups in a case of n = 3.

As shown in this result, it was found that even in a case where the active ingredient is suspended after administration, the initial release amount cannot be suppressed with hyaluronic acid. It was found that even in a case of a highly viscous preparation such as hyaluronic acid, since the hyaluronic acid is not gelated or precipitated at a physiological saline concentration, gelation and precipitation in situ are required for long-term sustained release as in the case of a hyaluronic acid derivative.

Subsequently, the following pharmacokinetic parameters were analyzed by noncompartmental analysis using WinNonlin Ver. 8.3 (manufactured by Pharsight Corporation) by the same method as in Example 1, and the values thereof are listed in Table 6.
· Average retention time (MRT), unit: hour
· Half-life (T1/2), unit: hour
· Maximum blood concentration (Cmax), unit: ng/mL

**[Table 8]**

| | T1/2 (h) | MRT (h) | Cmax (ng/mL) |
|---|---|---|---|
| Example 5-1 | 151.6 | 185.6 | 26.1 |
| Comparative Example 5-1 | 154.3 | 189.7 | 36.3 |
| Comparative Example 5-2 | 160.0 | 159.2 | 42.5 |

In a case where the sustained-release properties were evaluated by Expression (A), the sustained-release properties of Example 5-1 were 1.2 with respect to Comparative Example 5-1, and the sustained-release properties of Example 5-1 were 1.0 with respect to Comparative Example 5-2. Therefore, there was almost no difference in the sustained-release properties between the examples and the comparative examples because the sustained-release properties were high in all the above-described examples and comparative examples.

In a case where the initial burst was evaluated by Expression (B), it was found that the initial burst of Example 5-1 was 0.61 as compared with Comparative Example 5-1, and the initial burst of Example 5-1 was 0.72 as compared with Comparative Example 5-2, which were confirmed that the initial release amount abnormality could be reduced.

As shown in this result, it was found that in the intra-articular administration, both the long-term sustained release and the burst suppression are achieved only by the pharmaceutical composition using the hyaluronic acid derivative.

### <Example 6: Subcutaneous administration>

A hyaluronic acid derivative pharmaceutical composition was produced by the following method.

### [Preparation of cyclosporin A suspension]

A suspension of cyclosporin was obtained by the same method as in Example 1. In a case where the measurement was performed using a DLS device (manufactured by Otsuka Electronics Co., Ltd., ELSZ2000), the average particle diameter was 157 nm and the polydispersity index was 0.225. As a result of quantifying the concentration of cyclosporin under the following conditions, the concentration of cyclosporin was 64.7 mg/mL.

### (Reverse phase chromatography analysis conditions)

HPLC device: JASCO HPLC-EXTREMA

### [HPLC quantification conditions: reverse phase chromatography analysis conditions]

Column: Inert Sustain C18, particle diameter 5 µm × inner diameter 4.6 mm × length 150 mm
Column temperature: 40°C
Mobile phase: eluent A, 0.1% TFA/acetonitrile
Eluent B: 0.1% TFA/water
Ratio between mobile phases: eluent A/eluent B = 9/1
Flow rate: 1 mL/min
Injection volume: 30 µL
Detector: UV (210 nm)

### [Preparation of hyaluronic acid nanogel]

A hyaluronic acid nanogel (weight-average molecular weight: 35 kDa, introduction rate of cholesteryl group: 19%) in which a cholesteryl group was introduced was synthesized as described in Examples 1 and 2 of PCT International Publication No. WO2010/053140.

12 mg/mL of a 10% sucrose-containing aqueous solution containing the HA derivative (35k19) synthesized in [Preparation of hyaluronic acid nanogel] was mixed with the cyclosporin A suspension prepared in [Preparation of cyclosporin A suspension], and a 10% sucrose solution was further added thereto. In this manner, a hyaluronic acid derivative pharmaceutical composition of Example 6-1 having a CyA concentration of 10 mg/mL was produced. The prepared samples are listed in Table 9. The final sucrose concentration was 8.5%.

### <Comparative Example 6-1>

The cyclosporin A suspension prepared in [Preparation of cyclosporin A Suspension] described above was dissolved in a 10% sucrose aqueous solution containing 0.05% polysorbate at a proportion set such that the CyA concentration was 10 mg/mL, and a 10% sucrose solution was further added thereto in Comparative Example 6-1. The final sucrose concentration was 8.5%.

### <Comparative Example 6-2>

A powder of 35 kDa hyaluronic acid (manufactured by Bloomage Biotechnology Corporation) was weighed and dissolved in a 10% sucrose aqueous solution at a concentration of 12 mg/mL. The adjusted 10% sucrose aqueous solution containing 35 kDa hyaluronic acid, the cyclosporin A suspension prepared in [Preparation of cyclosporin A suspension], and the 10% sucrose aqueous solution were mixed at a proportion set such that the CyA concentration was 10 mg/mL and the hyaluronic acid concentration was 5 mg/mL in Comparative Example 6-2. The final sucrose concentration was 8.5%.

### <Comparative Example 6-3>

A powder of Poloxamer 338 (manufactured by Sigma-Aldrich Co., LLC) was weighed in a sterile vial and dissolved in a 10% sucrose aqueous solution at a concentration of 150 mg/mL. The adjusted 10% sucrose aqueous solution containing Poloxamer 338, the cyclosporin A suspension prepared in [Preparation of cyclosporin A suspension] described above and the 10% sucrose aqueous solution were mixed at a proportion set such that the CyA concentration was 10 mg/mL and the Poloxamer 338 concentration was 50 mg/mL in Comparative Example 6-3. The final sucrose concentration was 8.5%.

### <Pharmacokinetic test>

Each of the samples of Example 6-1 and Comparative Examples 6-1 to 6-3 prepared in Example 6 was subcutaneously administered to a normal rat (SD, 6 weeks old, male) at a dose of 25 mg/kg using a syringe having a 25 G needle.

**[Table 9]**

| | Form and concentration of CyA | 35k19 concentration |
|---|---|---|
| Example 6-1 | Suspension | 5 mg/mL |
| | 10 mg/mL | |
| Comparative Example 6-1 | Suspension | 0 mg/mL |
| | 10 mg/mL | |
| Comparative Example 6-2 | Suspension | 0 mg/mL |
| | 10 mg/mL | |
| Comparative Example 6-3 | Suspension | 0 mg/mL |
| | 10 mg/mL | |

After the administration, blood was collected from the jugular vein with a syringe treated with heparin over time, and EDTA-K₂ was added as a protease inhibitor.

The obtained blood was separated into plasma and measured by LC-MS/MS. FIG. 10 shows a change in the blood plasma concentration of cyclosporin at the time of administering various cyclosporin suspension preparations.

As shown in FIG. 10, in Comparative Example 6-1, Comparative Example 6-2, and Comparative Example 6-3, the initial release amount abnormality was confirmed. On the contrary, in Example 6-1, the initial release amount abnormality was not confirmed.

As shown in this result, it was found that even in a case where the active ingredient is suspended after administration, the initial release amount cannot be suppressed in a case of hyaluronic acid or temperature-responsive polymer Poloxamer. 50 mg/mL of Poloxamer 338 used in Example 6-3 is an additive used in the suspension preparation RECAMBYS (Janssen Pharmaceutical K.K.), and the concentration is also the same. Since these preparations are not precipitated at a physiological saline concentration, it has been shown that gelation and precipitation in situ are required for both initial burst suppression and long-term sustained release as in a case of the hyaluronic acid derivative.

Subsequently, the following pharmacokinetic parameters were analyzed by noncompartmental analysis using WinNonlin Ver. 8.3 (manufactured by Pharsight Corporation) by the same method as in Example 1, and the values thereof are listed in Table 10.
· Average retention time (MRT), unit: hour
· Half-life (T1/2), unit: hour
· Maximum blood concentration (Cmax), unit: ng/mL

**[Table 10]**

| | T1/2 (h) | MRT (h) | Cmax (ng/mL) |
|---|---|---|---|
| Example 6-1 | 485 | 760 | 56 |
| Comparative Example 6-1 | 287 | 288 | 212 |
| Comparative Example 6-2 | 695 | 897 | 191 |
| Comparative Example 6-3 | 443 | 253 | 388 |

As a result of evaluating the sustained-release properties by Expression (A), the sustained-release properties of Example 6-1 were 2.6 as compared with Comparative Example 6-1, Example 6-1 were 0.8 as compared with Comparative Example 6-2, and Example 6-1 were 3.0 as compared with Comparative Example 6-3, and it was found that the sustained-release properties of Example 6-1 were significantly longer than those of Comparative Example 6-1 and Comparative Example 6-3.

In a case where the initial burst was evaluated by Expression (B), it was found that the initial burst of Example 6-1 was 0.26 as compared with Comparative Example 6-1, the initial burst of Example 6-1 was 0.29 as compared with Comparative Example 6-2, and the initial burst of Example 6-1 was 0.14 as compared with Comparative Example 6-3, which were confirmed that the initial release amount abnormality could be reduced by using the hyaluronic acid derivative.

### <Example 7>

A hyaluronic acid derivative pharmaceutical composition of itraconazole was produced by the following method.

2.5 mg of itraconazole ((manufactured by Tokyo Chemical Industry Co., Ltd.) was added to a 96-well plate, and 0.05 mL of 0.01% polysorbate 80 was added thereto. Further, 200 mg of ZrO₂ beads (manufactured by Nikkato Corporation) having a diameter of 0.2 mm were added thereto, and 0.05 mL of 1% polysorbate 80 was added thereto. The rotor was placed therein, and the mixture was stirred with a magnetic stirrer for 3 days and finely pulverized. A part of the dispersion liquid was taken out, diluted 200 times with 0.01% polysorbate 80, and measured using a DLS device (manufactured by Otsuka Electronics Co., Ltd., ELSZ2000). As a result, the average particle diameter thereof was 371 nm and the polydispersity index thereof was 0.21. 0.05 mL of a 12 mg/mL aqueous solution of the HA derivative (35k19) synthesized in Example 1 [Preparation of hyaluronic acid nanogel] was mixed with 0.05 mL of the itraconazole suspension to produce a hyaluronic acid derivative pharmaceutical composition of itraconazole. In a case where this pharmaceutical composition was added dropwise to a subcutaneous simulated solution (PBS solution containing 0.8% BSA (bovine serum albumin)), it was confirmed that the hyaluronic acid derivative pharmaceutical composition was gelated.

The precipitate could be visually observed the next day, and the shape of the gel was maintained even after being shaken.

### <Logical Example 1>

A hyaluronic acid derivative pharmaceutical composition of macrocyclic peptide MK-0616 (manufactured by Merck KGaA, molecular weight: 1616.0) can be produced by the following method.

720 mg of MK-0616 is weighed in a 30 mL glass vial, and 14.4 mL of 0.01% polysorbate 80 is added thereto. Further, 10 g of 0.2 mm ZrO₂ beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of MK-0616 is taken out with a syringe, placed in a 15 mL conical tube, and centrifuged at 10,000 g for 60 minutes, and the supernatant is discarded.

Further, 10 times the amount of water for injection is added thereto, and the mixture is centrifuged at 10,000 g for 60 minutes, and the supernatant is discarded, thereby obtaining a suspension of MK-0616. 1.0 mL of a 12 mg/mL aqueous solution of the HA derivative (35k19) synthesized in [Preparation of hyaluronic acid nanogel] of Example 1 is mixed with 1.0 mL of the MK-0616 suspension, thereby producing a hyaluronic acid derivative pharmaceutical composition of MK-0616. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 2>

A hyaluronic acid derivative pharmaceutical composition containing insulin crystals (molecular weight: 5,800) can be produced by the following method.

A crystalline insulin zinc aqueous suspension injection is obtained according to the Japanese Pharmacopoeia (14th edition). The crystalline insulin zinc aqueous suspension injection may be prepared according to Galenics of Insulin, Jens Brange, Springer Verlag, 1987 instead of the method described in the Japanese Pharmacopoeia (14th edition). 1.0 mL of a 12 mg/mL aqueous solution of the HA derivative (35k19) synthesized in [Preparation of hyaluronic acid nanogel] of Example 1 is mixed with 1.0 mL of the crystalline insulin zinc aqueous suspension injection solution, thereby producing a hyaluronic acid derivative pharmaceutical composition of insulin crystals. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 3>

A hyaluronic acid derivative pharmaceutical composition of carbamazepine-saccharin cocrystal (molecular weight of carbamazepine: 236.3) can be produced by the following method.

A carbamazepine-saccharin cocrystal is obtained by the method described in Published Japanese Translation No. 2007-507554 of the PCT International Publication. 720 mg of the carbamazepine-saccharin cocrystal is weighed in a 30 mL glass vial, and 14.4 mL of 0.01% polysorbate 80 is added thereto. Further, 10 g of 0.2 mm ZrO₂ beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of the carbamazepine-saccharin cocrystal is taken out with a syringe, placed in a 15 mL conical tube, and centrifuged at 10,000 g for 60 minutes, and the supernatant is discarded. Further, 10 times the amount of water for injection is added thereto, and the mixture is centrifuged at 10,000 g for 60 minutes, and the supernatant is discarded to obtain a carbamazepine-saccharin suspension. 1.0 mL of a 12 mg/mL aqueous solution of the HA derivative (35k19) synthesized in [Preparation of hyaluronic acid nanogel] of Example 1 is mixed with 1.0 mL of the carbamazepine-saccharin cocrystal suspension, thereby producing a hyaluronic acid derivative pharmaceutical composition of carbamazepine-saccharin. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 4>

A hyaluronic acid derivative pharmaceutical composition of itraconazole (molecular weight: 705.6) can be produced by the following method.

720 mg of itraconazole (manufactured by Tokyo Chemical Industry Co., Ltd.) is weighed in a 30 mL glass vial, and 14.4 mL of 0.01% polysorbate 80 is added thereto. Further, 10 g of 0.2 mm ZrO2 beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of itraconazole is taken out with a syringe, placed in a 15 mL conical tube, and centrifuged at 10,000 g for 60 minutes, and the supernatant is discarded. Further, 10 times the amount of water for injection is added thereto, and the mixture is centrifuged at 10,000 g for 60 minutes, and the supernatant is discarded to obtain a suspension of itraconazole. 3.0 mL of a 40 mg/mL aqueous solution of the HA derivative (10k30) synthesized in Example 2 was mixed with 1.0 mL of the itraconazole suspension, thereby producing a hyaluronic acid derivative pharmaceutical composition of itraconazole. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 5>

A hyaluronic acid derivative pharmaceutical composition of macrocyclic peptide MK-0616 (manufactured by Merck KGaA, molecular weight: 1616.0) can be produced by the following method.

720 mg of MK-0616 is weighed in a 30 mL glass vial, and 14.4 mL of 0.01% polysorbate 80 is added thereto. Further, 10 g of 0.2 mm ZrO₂ beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of MK-0616 is taken out with a syringe, placed in a 15 mL conical tube, and centrifuged at 10,000 g for 60 minutes, and the supernatant is discarded. Further, 10 times the amount of water for injection is added thereto, and the mixture is centrifuged at 10,000 g for 60 minutes, and the supernatant is discarded, thereby obtaining a suspension of MK-0616. 3.0 mL of a 40 mg/mL aqueous solution of the HA derivative (10k30) synthesized in [Preparation of hyaluronic acid nanogel] of Example 2 is mixed with 1.0 mL of the MK-0616 suspension, thereby producing a hyaluronic acid derivative pharmaceutical composition of MK-0616. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 6>

A hyaluronic acid derivative pharmaceutical composition containing insulin crystals (molecular weight: 5,800) can be produced by the following method.

A crystalline insulin zinc aqueous suspension injection is obtained according to the Japanese Pharmacopoeia (14th edition). The crystalline insulin zinc aqueous suspension injection may be prepared according to Galenics of Insulin, Jens Brange, Springer Verlag, 1987 instead of the method described in the Japanese Pharmacopoeia (14th edition). 3.0 mL of a 40 mg/mL aqueous solution of the HA derivative (10k30) synthesized in [Preparation of hyaluronic acid nanogel] of Example 2 is mixed with 1.0 mL of a crystalline insulin zinc aqueous suspension injection solution, thereby producing a hyaluronic acid derivative pharmaceutical composition of insulin crystals. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 7>

A hyaluronic acid derivative pharmaceutical composition of carbamazepine-saccharin cocrystal (molecular weight of carbamazepine: 236.3) can be produced by the following method.

A carbamazepine-saccharin cocrystal is obtained according to Published Japanese Translation No. 2007-507554 of the PCT International Publication. 720 mg of the carbamazepine-saccharin cocrystal is weighed in a 30 mL glass vial, and 14.4 mL of 0.01% polysorbate 80 is added thereto. Further, 10 g of 0.2 mm ZrO2 beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of the carbamazepine-saccharin cocrystal is taken out with a syringe, placed in a 15 mL conical tube, and centrifuged at 10,000 g for 60 minutes, and the supernatant is discarded. Further, 10 times the amount of water for injection is added thereto, and the mixture is centrifuged at 10,000 g for 60 minutes, and the supernatant is discarded to obtain a carbamazepine-saccharin suspension. 3.0 mL of a 40 mg/mL aqueous solution of the HA derivative (10k30) synthesized in [Preparation of hyaluronic acid nanogel] of Example 2 is mixed with 1.0 mL of the carbamazepine-saccharin cocrystal suspension, thereby producing a hyaluronic acid derivative pharmaceutical composition of carbamazepine-saccharin. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 8>

A hyaluronic acid derivative pharmaceutical composition of acetaminophen (manufactured by Tokyo Chemical Industry Co., Ltd., molecular weight: 151.2) can be produced by the following method.

720 mg of acetaminophen is weighed in a 30 mL glass vial, and 14.4 mL of 0.01% polysorbate 80 is added thereto. Further, 10 g of 0.2 mm ZrO₂ beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of acetaminophen is taken out with a syringe, placed in a 15 mL conical tube, and centrifuged at 10,000 g for 60 minutes, and the supernatant is discarded.

Further, 10 times the amount of water for injection is added thereto, and the mixture is centrifuged at 10,000 g for 60 minutes, and the supernatant is discarded to obtain a suspension of acetaminophen. 1.0 mL of a 12 mg/mL aqueous solution of the HA derivative (35k19) synthesized in Example 1 [Preparation of hyaluronic acid nanogel] is mixed with 1.0 mL of the 1.0 mL of the acetaminophen suspension, thereby producing a hyaluronic acid derivative pharmaceutical composition of acetaminophen. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### <Logical Example 9>

A hyaluronic acid derivative pharmaceutical composition of acetaminophen (manufactured by Tokyo Chemical Industry Co., Ltd., molecular weight: 151.2) can be produced by the following method.

720 mg of acetaminophen is weighed in a 30 mL glass vial, and 14.4 mL of 0.01% polysorbate 80 is added thereto. Further, 10 g of 0.2 mm ZrO₂ beads (manufactured by Nikkato Corporation) are added thereto, and 14.4 mL of 1% polysorbate 80 is added thereto. The rotor is placed therein, and the mixture is stirred with a magnetic stirrer for 3 days and finely pulverized. The suspension of acetaminophen is taken out with a syringe, placed in a 15 mL conical tube, and centrifuged at 10,000 g for 60 minutes, and the supernatant is discarded.

Further, 10 times the amount of water for injection is added thereto, and the mixture is centrifuged at 10,000 g for 60 minutes, and the supernatant is discarded to obtain a suspension of acetaminophen. 3.0 mL of a 40 mg/mL aqueous solution of the HA derivative (10k30) synthesized in [Preparation of hyaluronic acid nanogel] of Example 2 is mixed with 1.0 mL of the acetaminophen suspension, thereby producing a hyaluronic acid derivative pharmaceutical composition of acetaminophene. In a case where this pharmaceutical composition is added dropwise to a subcutaneous simulated solution (PBS solution containing 0.8% BSA (bovine serum albumin)), it can be sufficiently presumed that this hyaluronic acid derivative pharmaceutical composition is gelated and precipitated.

### INDUSTRIAL APPLICABILITY

According to the hyaluronic acid derivative pharmaceutical composition of the present embodiment, it is possible to provide a hyaluronic acid derivative pharmaceutical composition which maintains a concentration of an active ingredient in vivo for a long period of time and reduces an initial release amount abnormality (initial burst).

## Claims

1. A hyaluronic acid derivative pharmaceutical composition comprising:
an active ingredient suspended in an aqueous carrier containing a hyaluronic acid derivative,
wherein the active ingredient is a fine particle having a molecular weight of 100 g/mol or greater and 8000 g/mol or less.

2. The hyaluronic acid derivative pharmaceutical composition according to Claim 1,
wherein the fine particle is a microparticle or a nanoparticle.

3. The hyaluronic acid derivative pharmaceutical composition according to Claim 1 or 2,
wherein the hyaluronic acid derivative has one or more repeating units represented by General Formula (I), 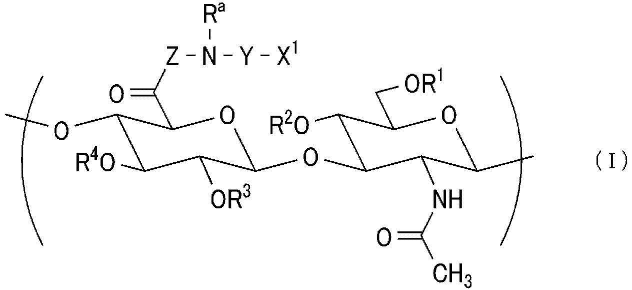
(in the formula, R¹, R², R³, and R⁴ each independently represent a group selected from the group consisting of a hydrogen atom, C₁₋₆ alkyl, formyl, and C₁₋₆ alkylcarbonyl,
Z represents a direct bond or a peptide linker formed of 2 or more and 30 or less of any amino acid residues,
X¹ represents a group selected from the group consisting of a group represented by -NR^{b}-R, -NR^{b}-COO-R, -NR^{b}-CO-R, -NR^{b}-CO-NR^{c}-R, -COO-R, -O-COO-R, -S-R, - CO-Y^{a}-S-R, -O-CO-Y^{b}-S-R, -NR^{b}-CO-Y^{b}-S-R, and -S-S-R,
R^{a}, R^{b}, and R^{c} each independently represent a group selected from the group consisting of a hydrogen atom, C₁₋₂₀ alkyl, amino C₂₋₂₀ alkyl, and hydroxy C₂₋₂₀ alkyl,
a group selected from the group consisting of -O- and -NR^{f}- may be inserted into an alkyl moiety of each of R^{a}, R^{b}, and R^{c},
R^{f} represents a group selected from the group consisting of a hydrogen atom, C₁₋₁₂ alkyl, amino C₂₋₁₂ alkyl, and hydroxy C₂₋₁₂ alkyl,
a group selected from the group consisting of -O- and -NH- may be inserted into an alkyl moiety of R^{f},
R represents a hydrophobic group or a hydrophilic group,
Y represents C₂₋₃₀ alkylene or -(CH₂CH₂O)ₘ-CH₂CH₂-,
where a group selected from the group consisting of -O-, -NR^{g}-, and -S-S- may be inserted into alkylene of Y,
R^{g} represents a group selected from the group consisting of a hydrogen atom, C₁₋₂₀ alkyl, amino C₂₋₂₀ alkyl, and hydroxy C₂₋₂₀ alkyl,
a group selected from the group consisting of -O- and -NH- may be inserted into an alkyl moiety of R^{g},
Y^{a} represents C₁₋₅ alkylene,
Y^{b} represents C₂₋₈ alkylene or C₂₋₈ alkenylene, and
m represents an integer of 1 or greater and 100 or less).

4. The hyaluronic acid derivative pharmaceutical composition according to Claim 3,
wherein R represents a hydrophobic group.

5. The hyaluronic acid derivative pharmaceutical composition according to Claim 4,
wherein the hydrophobic group is a cholesteryl group.

6. The hyaluronic acid derivative pharmaceutical composition according to Claim 1 or 2,
wherein the hyaluronic acid derivative is precipitated or salted out in vivo or under a physiological condition.

7. The hyaluronic acid derivative pharmaceutical composition according to Claim 1 or 2,
wherein the active ingredient is a poorly water-soluble drug.

8. The hyaluronic acid derivative pharmaceutical composition according to Claim 1 or 2,
wherein the active ingredient is a cyclic peptide.

9. The hyaluronic acid derivative pharmaceutical composition according to Claim 8,
wherein the cyclic peptide contains 4 to 49 amino acids in terms of a cyclic size.

10. The hyaluronic acid derivative pharmaceutical composition according to Claim 8,
wherein the cyclic peptide contains an unnatural amino acid.

11. The hyaluronic acid derivative pharmaceutical composition according to Claim 8,
wherein the number of kinds of amino acids constituting the cyclic peptide is 8 or more and 20 or less.

12. The hyaluronic acid derivative pharmaceutical composition according to Claim 1 or 2,
wherein the hyaluronic acid derivative pharmaceutical composition is administered subcutaneously, intramuscularly, intraspinally, intraarticularly, intracerebrally, intravitreally, or to an ocular surface, a nose, a lung, or a mouth as an administration site.

13. The hyaluronic acid derivative pharmaceutical composition according to Claim 1 or 2,
wherein an administration method is injection, eye drop, or application.
